# EUROPEAN PATENT APPLICATION

(11) **EP 4 799 544 A1**
(43) Date of publication of application: **02.09.2026**
(21) Application number: 26160235.3
(22) Date of filing: 24.02.2026
(51) Int. Cl.: A47L 11/40

(54) **ROBOT CLEANER AND CONTROLLING METHOD OF THE SAME**

(30) Priority: 26.02.2025 KR 20250025319
(71) Applicant: LG Electronics Inc., Yeongdeungpo-gu Seoul 07336 (KR)
(72) Inventor: LEE, Dongjae, 08592 Seoul (KR); HAN, Seungwoo, 08592 Seoul (KR); KWON, HyeonJung, 08592 Seoul (KR)
(74) Representative: Vossius & Partner Patentanwälte Rechtsanwälte mbB

(57) **Abstract**

The present invention relates to a method of controlling a robot cleaner station (100), the method including a mop washing step of washing a mop (242) of a robot cleaner (200) by supplying washing water to the mop in a state in which the robot cleaner is coupled to the robot cleaner station, a sterilizing/washing step of sterilizing the mop by discharging heated water or wet vapor to the mop, and a mop drying step of drying the mop by operating a blower fan (175) configured to discharge air toward the mop, in which the blower fan operates in the sterilizing/washing step, thereby preventing an increase in internal humidity of the robot cleaner station and improving efficiency of a drying process.

## Description

### [Technical Field]

The present invention relates to a robot cleaner station, and more particularly, to a built-in robot cleaner station to which a robot cleaner is coupled, such that when the robot cleaner is coupled to the built-in station, the built-in station may collect dust from a dust bin of the robot cleaner, wash a mop of the robot cleaner, and dry the mop, and a method of controlling the same.

### [Background Art]

Recently, with the advancement of industrial technologies, robot cleaners, which clean zones required to be cleaned while autonomously traveling without user operations, have been developed.

The robot cleaner may be equipped with a sensor capable of recognizing a space to be cleaned, an agitator capable of cleaning and sweeping a floor surface, and a mop or the like capable of wiping the floor surface. The robot cleaner may travel while sucking dust from the floor surface in the space recognized by the sensor and wiping the floor surface with the mop or the like.

Among the robot cleaners, there are a dry robot cleaner capable of sucking and removing debris scattered on the floor surface, and a wet robot cleaner capable of wiping the floor surface with the mop containing moisture in order to effectively remove debris attached to the floor surface. The dry robot cleaner is equipped with a dust bin and sucks debris on the floor surface by using a suction force of a suction motor. The wet robot cleaner is equipped with a water container and is configured such that water accommodated in the water container is supplied to the mop and the mop containing moisture wipes the floor surface to effectively remove debris attached to the floor surface. In addition, there is also a robot cleaner equipped with both the agitator and the mop.

A charging stand for the robot cleaner refers to a device in which the robot cleaner, which has completed a cleaning process, is docked. The charging stand is configured to charge a battery provided in the robot cleaner by supplying electric power to the battery. The charging stand is equipped with a power supply module therein. The charging stand is equipped with a charging terminal connected to the power supply module, and the robot cleaner is equipped with a corresponding terminal. In case that the charging terminal and the corresponding terminal come into contact with each other, electric power is supplied to the battery, and the battery is charged.

Meanwhile, in case that the charging stand for the robot cleaner is disposed in a building, the charging stand occupies a predetermined range of an indoor space. In this case, spatial efficiency of the interior may deteriorate. In addition, there may be a risk that the robot cleaner may collide with a user or pet while the user or pet is moving, which causes injury or damage to both the user or pet and the robot cleaner.

In addition, in the case of a station to which a dust collection function of the robot cleaner is added, there is a limitation in that a volume occupied by the station increases, which may degrade the aesthetics of the interior.

Meanwhile, Chinese Utility Model Registration No. CN 218922468 U discloses a cleaner station in which a robot cleaner is coupled to a lower side of a washing machine, the robot cleaner is charged, dust is collected from the robot cleaner, and a wet mop of the robot cleaner is washed.

The cleaner station is configured to dry the mop, which is disposed above a washing tub, by supplying dry air to the washing tub in which the mop of the robot cleaner is washed.

However, in case that the mop is dried in an open space of an interior of the cleaner station, there is a limitation in that interior humidity may be increased by water vapor generated during the drying process, and an offensive odor, which is generated while wastewater generated during the process of washing the mop is evaporated, may spread into the interior.

In addition, because heated air spreads to the outside during the process of drying the mop in an open space, heated air needs to be continuously supplied over a long period of time, which causes a limitation in that energy efficiency deteriorates.

In addition, in the case of a station that dries a mop by supplying steam, the occurrence of offensive odors and sanitation-related problems may be caused when inside moisture and contaminants are not appropriately removed after the mop is washed.

### [Disclosure]

### [Technical Problem]

The present invention is proposed to solve these problems of the robot cleaner station in the related art and aims to provide a robot cleaner station capable of being built-in below a kitchen cabinet without requiring a separate installation space.

The present invention also aims to provide a robot cleaner station capable of accommodating a robot cleaner in a lower space of a kitchen cabinet having a predetermined height limit.

The present invention also aims to provide a robot cleaner station capable of automatically collecting dust from an interior of a dust bin of a robot cleaner when the robot cleaner is coupled.

The present invention also aims to provide a robot cleaner station capable of appropriately adjusting humidity inside the robot cleaner station to solve sanitation-related problems that may occur during a process of washing and drying a mop, and a method of controlling the same.

### [Technical Solution]

In order to achieve the above-mentioned objects, a robot cleaner station according to the present invention may include: a housing including an upper cover; a base accommodated in the housing and disposed below a robot cleaner; an accommodation space formed between the base and the upper cover and configured to accommodate the robot cleaner; a mop washing part configured to wash a mop of the robot cleaner; and a mop drying part configured to dry the mop by discharging air to the mop, in which the mop washing part includes a steam supply part configured to heat water and supply hot water or wet vapor to the accommodation space, in which the mop drying part includes a blower fan configured to generate an airflow and discharge the air into the accommodation space, and in which the blower fan is configured to operate while the steam supply part operates.

Meanwhile, the robot cleaner station may include: an air discharge part configured to discharge air from the accommodation space, in which the air discharge part includes: an air suction port configured to communicate with the accommodation space and suck air; and an air discharge fan configured to provide a flow force to the air sucked through the air suction port, and in which the air discharge fan is configured to operate while the steam supply part operates.

In addition, the blower fan may be disposed in the housing so that a side of the blower fan at which air is discharged is directed toward the accommodation space, and a side of the air discharge fan at which air is sucked may be disposed to be directed toward the accommodation space.

Meanwhile, the housing may include a door configured to open or close an inlet/outlet through which the robot cleaner enters or exits, and the door may be configured to be opened while the steam supply part operates.

In addition, the robot cleaner station may further include: a humidity sensor configured to measure humidity in the housing, in which the air discharge fan operates when the humidity measured by the humidity sensor is equal to or greater than a predetermined reference value.

Meanwhile, the robot cleaner station may further include: a humidity sensor configured to measure humidity in the housing, in which the blower fan operates when the humidity measured by the humidity sensor is equal to or greater than a predetermined reference value.

Meanwhile, the robot cleaner station may further include: a humidity sensor configured to measure humidity in the housing, in which the door is opened when the humidity measured by the humidity sensor is equal to or greater than a predetermined reference value.

A method of controlling a robot cleaner station according to the present invention may include: a mop washing step of washing a mop of a robot cleaner by supplying washing water to the mop in a state in which the robot cleaner is coupled to the robot cleaner station; a sterilizing/washing step of sterilizing the mop by discharging heated water or wet vapor to the mop; and a mop drying step of drying the mop by operating a blower fan configured to discharge air toward the mop, in which the blower fan operates in the sterilizing/washing step.

In addition, the water may be heated to 55°C or more and less than 70°C in the sterilizing/washing step.

In addition, the water may be heated to 100°C or more in the sterilizing/washing step.

In addition, an air discharge fan configured to discharge air from an interior of the robot cleaner station may operate in the sterilizing/washing step.

In addition, air may be introduced when humidity in the robot cleaner station is equal to or greater than a predetermined reference value.

Meanwhile, a door of the robot cleaner station may be opened when humidity in the robot cleaner station is equal to or greater than a predetermined reference value.

Meanwhile, the blower fan may operate when humidity in the robot cleaner station is equal to or greater than a predetermined reference value.

In addition, the introduction of the air may be stopped when the humidity in the robot cleaner station is less than the predetermined reference value.

In addition, the door may be closed when the humidity in the robot cleaner station is less than the predetermined reference value.

In addition, the operation of the blower fan may be stopped when the mop drying step ends.

Meanwhile, the operation of the air discharge fan may be stopped when the mop drying step ends.

### [Advantageous Effects]

According to the robot cleaner station according to the present invention described above, the module, which may charge the robot cleaner, collect dust, and wash the mop, may be disposed in the direction parallel to the robot cleaner, such that the lower space of the kitchen cabinet may be utilized.

In addition, the charging terminal, the dust collection part, the mop washing part, and the mop drying part are disposed to be surrounded based on the robot cleaner, such that various functions of the robot cleaner may be performed.

In addition, the surfaces, which exclude the front surface, may be covered by the kitchen cabinet, thereby providing the user with an aesthetic interior appearance.

In addition, even during the mop washing process using water or steam, an increase in internal humidity may be prevented, and efficiency of the drying process may be improved.

### [Description of Drawings]

FIG. 1 is a view for explaining a state in which a cleaner system according to an embodiment of the present invention is installed below a kitchen cabinet.
FIG. 2 is a view for explaining a relationship in which a pipe of the cleaner system according to the embodiment of the present invention is connected to a water drain pipe.
FIG. 3 is a perspective view for explaining the cleaner system according to the embodiment of the present invention.
FIG. 4 is a top plan view of FIG. 3.
FIG. 5 is a perspective view for explaining a robot cleaner according to the embodiment of the present invention.
FIG. 6 is a side view of FIG. 5.
FIG. 7 is a bottom plan view of FIG. 5.
FIG. 8 is a rear view of FIG. 5.
FIG. 9 is a perspective view for explaining a structure of a robot cleaner station according to the embodiment of the present invention.
FIG. 10 is a perspective view for explaining a door part of the robot cleaner station according to the embodiment of the present invention.
FIG. 11 is a perspective view for explaining an internal structure of the robot cleaner station according to the embodiment of the present invention.
FIG. 12 is a top plan view for explaining a mop drying part of the robot cleaner station according to the embodiment of the present invention.
FIG. 13 is a cross-sectional view taken along line A-A in FIG. 12.
FIG. 14 is a cross-sectional view taken along line B-B in FIG. 12.
FIG. 15 is a view for explaining a state in which an upper cover is separated from the robot cleaner station according to the embodiment of the present invention.
FIG. 16 is a block diagram for explaining a control configuration of the present invention.
FIGS. 17 to 19 are flowcharts for explaining a method of controlling the robot cleaner station according to the embodiment of the present invention.
FIG. 20 is a flowchart for explaining a method of controlling the robot cleaner station according to another embodiment of the present invention.
FIG. 21 is a view for explaining operations of a steam supply part, a heater, a blower fan, and an air discharge fan over time in the method of controlling the robot cleaner station according to the embodiment of the present invention.

### [Mode for Invention]

Hereinafter, exemplary embodiments of the present invention will be described in detail with reference to the accompanying drawings.

The present invention may be variously modified and may have various embodiments, and particular embodiments illustrated in the drawings will be specifically described below. The description of the embodiments is not intended to limit the present invention to the particular embodiments, but it should be interpreted that the present invention is to cover all modifications, equivalents and alternatives falling within the spirit and technical scope of the present invention.

In the description of the present invention, the terms such as "first" and "second" may be used to describe various constituent elements, but the constituent elements may not be limited by the terms. These terms are used only to distinguish one constituent element from another constituent element. For example, a first component may be named a second component, and similarly, the second component may also be named the first component, without departing from the scope of the present invention.

The term "and/or" may include any and all combinations of a plurality of the related and listed items.

When one constituent element is described as being "coupled" or "connected" to another constituent element, it should be understood that one constituent element can be coupled or connected directly to another constituent element, and an intervening constituent element can also be present between the constituent elements. When one constituent element is described as being "coupled directly to" or "connected directly to" another constituent element, it should be understood that no intervening constituent element is present between the constituent elements.

The terminology used herein is used for the purpose of describing particular embodiments only and is not intended to limit the present invention. Singular expressions may include plural expressions unless clearly described as different meanings in the context.

The terms "comprises," "comprising," "includes," "including," "containing," "has," "having" or other variations thereof are inclusive and therefore specify the presence of stated features, integers, steps, operations, elements, components, and/or combinations thereof, but do not preclude the presence or addition of one or more other features, integers, steps, operations, elements, components, and/or combinations thereof.

Unless otherwise defined, all terms used herein, including technical or scientific terms, may have the same meaning as commonly understood by those skilled in the art to which the present invention pertains. The terms such as those defined in a commonly used dictionary may be interpreted as having meanings consistent with meanings in the context of related technologies and may not be interpreted as ideal or excessively formal meanings unless explicitly defined in the present application.

Further, the following embodiments are provided to more completely explain the present invention to those skilled in the art, and shapes and sizes of elements illustrated in the drawings may be exaggerated for a more apparent description.

### KITCHEN CABINET AND CLEANER SYSTEM

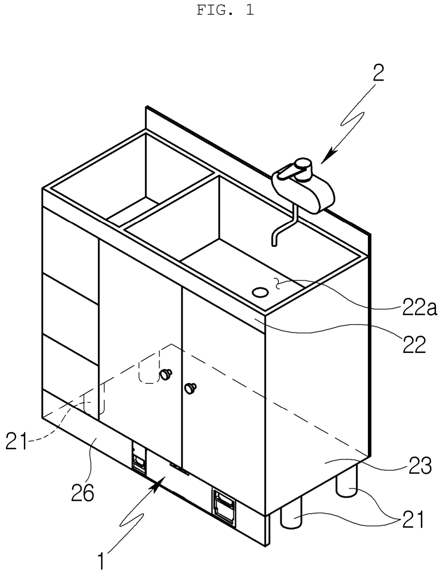

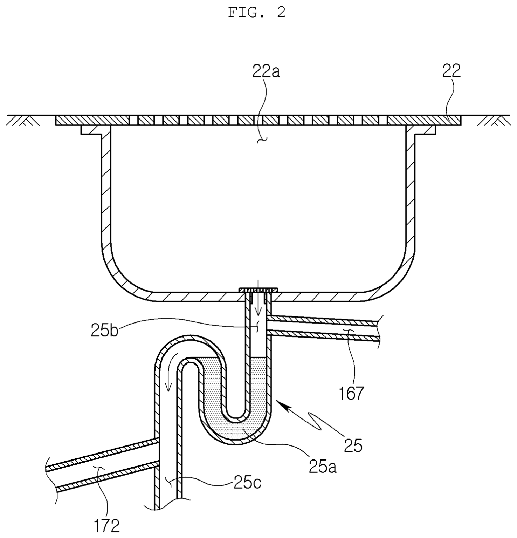

FIG. 1 is a view for explaining a state in which a cleaner system according to an embodiment of the present invention is installed below a kitchen cabinet, and FIG. 2 is a view for explaining a relationship in which a pipe of the cleaner system according to the embodiment of the present invention is connected to a water drain pipe.

With reference to FIGS. 1 and 2, a cleaner system 1 according to an embodiment of the present invention may be provided below a kitchen cabinet 2. Specifically, the kitchen cabinet 2 may be disposed in a kitchen and provide a space in which dishes, plates, cups, and the like may be accommodated and food preparation or dishwashing may be performed.

In addition, the kitchen cabinet 2 may be equipped with an upper plate (worktop) capable of serving as a sink, a cooking stage, or a working stage.

For example, the kitchen cabinet 2 may include a sink configured to provide a space in the upper plate so that dishwashing may be performed. Alternatively, the kitchen cabinet 2 may include a cooking stage on which food preparation is performed. In addition, the kitchen cabinet 2 may include a gas range stage on the upper plate on which a gas range, an induction cooktop, a halogen hob, an oven, or the like is installed.

In general, a standard cabinet having a width of 600 mm in a forward/rearward direction and a width of 600 mm in a leftward/rightward direction may be used as the kitchen cabinet 2.

The cleaner system 1 according to another embodiment of the present invention may be provided below a structure including at least any one of a water supply pipe and a water drain pipe. Specifically, the water supply pipe may refer to a flow path connected to an external water supply source configured to supply a fluid to the structure, and the water drain pipe may refer to a flow path configured to discharge a fluid, which is discharged from the structure, to a sewage system.

A stomope cabinet configured to store dishes, kitchen tools, and the like may be provided below the kitchen cabinet 2 or the structure. That is, the kitchen cabinet 2 or the structure may include an upper plate 22 configured to provide a space in which tasks such as cooking or dishwashing may be performed, a lower plate 23 disposed to be spaced apart from a ground surface by a predetermined height, and an accommodation space formed between the upper plate 22 and the lower plate 23 and configured to accommodate dishes, kitchen tools, and the like. In this case, in case that the kitchen cabinet 2 is a sink, a basin 22a may be disposed in the upper plate 22.

In addition, the lower plate 23 may be supported by pedestals 21. The pedestals 21 may be disposed in a direction perpendicular to a floor of the kitchen and support a load of the kitchen cabinet 2. In this case, a space may be formed between the floor of the kitchen and the lower plate 23 in accordance with heights of the pedestals 21.

On the contrary, the kitchen cabinet 2 may be fixed to a wall of a building without the pedestal 21. Even in this case, a space may be formed between the floor of the kitchen and the lower plate 23.

The cleaner system 1 according to the embodiment of the present invention is mounted in a space (hereinafter, referred to as a 'mounting space') between the floor of the kitchen and the lower plate 23, as described above.

For example, the mounting space may have a height of 200 mm or less, and generally, a height of 160 mm or less.

Therefore, according to the present invention, the cleaner system 1 is disposed in a lower space of the kitchen cabinet 2, which minimizes a degree to which the cleaner system 1 is exposed to the outside.

In addition, the cleaner system 1 does not occupy a separate space in comparison with a case in which a charging stand for a robot cleaner is disposed in a predetermined space of a living room, a room, or a kitchen. The cleaner system 1 is disposed in an unused space formed by the kitchen cabinet 2, thereby maximizing spatial efficiency.

Meanwhile, the kitchen cabinet 2 or the structure is equipped with a water drain pipe 25 capable of draining liquids used for cooking or water used for dishwashing. At least a part of the water drain pipe 25 may be disposed in the accommodation space formed between the upper plate 22 and the lower plate 23. In general, the water drain pipe 25 may be connected to a drain port formed in the basin 22a of the sink. The water drain pipe 25 includes a U-trap 25a configured to prevent a reverse flow of contaminated gas or offensive odor. The U-trap 25a may be disposed in the accommodation space. A liquid introduced through the drain port flows downward by gravity at an upstream side 25b of the U-trap and accumulates in the U-trap 25a. When the liquid accumulates so that a level of the liquid becomes equal to or higher than a preset predetermined level by the U-trap 25a, the liquid may flow downward along a downstream side 25c of the U-trap and be discharged to the sewage system.

The cleaner system 1 according to the embodiment of the present invention may wash and dry mops 242 of a robot cleaner 200 by using the above-mentioned water drain pipe 25.

In addition, although not illustrated, the kitchen cabinet 2 may be equipped with the water supply pipe. Fresh water (or purified water) may be supplied to the cleaner system 1 through the water supply pipe.

Hereinafter, a specific structure of the cleaner system 1 will be described.

### CLEANER SYSTEM

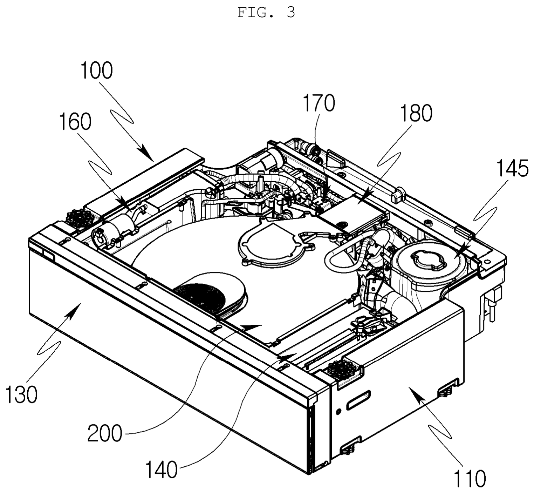

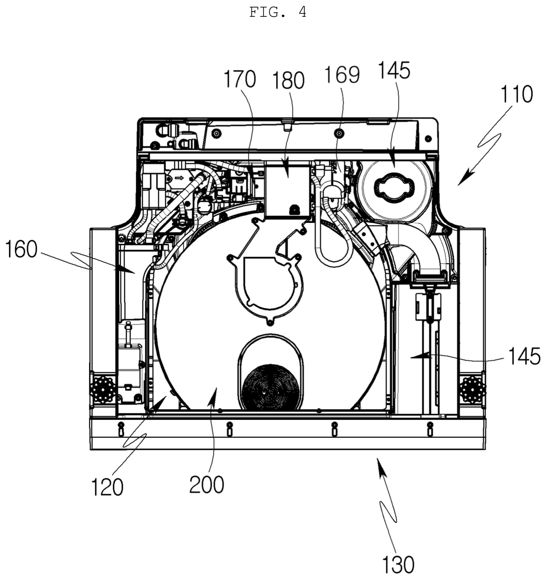

Meanwhile, FIG. 3 is a perspective view for explaining the cleaner system according to the embodiment of the present invention. FIG. 4 is a top plan view of FIG. 3.

The cleaner system 1 according to the embodiment of the present specification may include a robot cleaner station 100 and the robot cleaner 200.

The cleaner system 1 includes the robot cleaner station 100. The robot cleaner 200 may be coupled to the robot cleaner station 100. Specifically, the robot cleaner 200 may enter a front side of the robot cleaner station 100, and the robot cleaner 200 may be accommodated in the robot cleaner station 100. The robot cleaner station 100 may remove dust from a dust bin 220 of the robot cleaner 200. The robot cleaner station 100 may wash a rotary cleaning part 240 of the robot cleaner 200. The robot cleaner station 100 may dry the rotary cleaning part 240 of the robot cleaner 200. The robot cleaner station 100 may supply electric power to the robot cleaner 200.

### ROBOT CLEANER

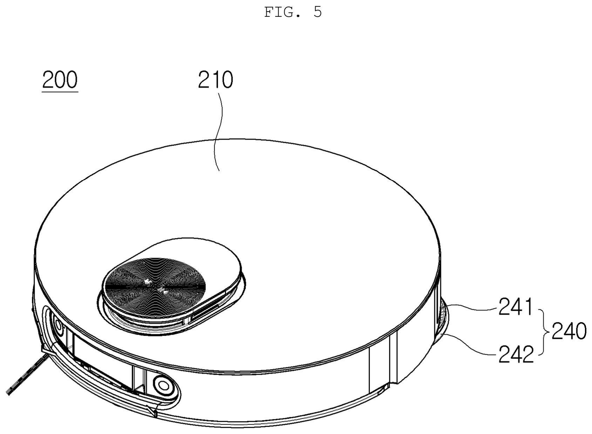

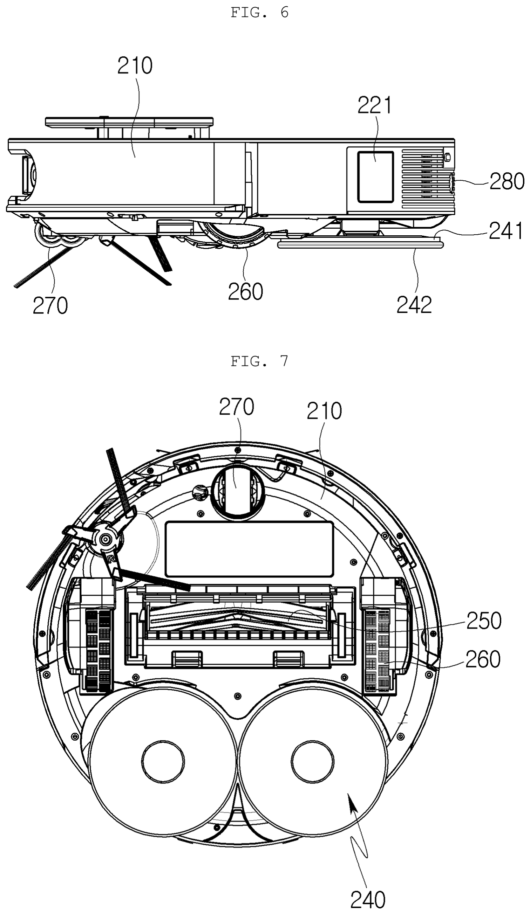

Meanwhile, FIGS. 5 to 8 are views for explaining the robot cleaner in the robot cleaner system according to the embodiment of the present invention.

A structure of a robot cleaner 200 will be described below with reference to FIGS. 5 to 8.

The robot cleaner 200 may automatically clean a zone to be cleaned by sucking foreign substances such as dust from a floor while autonomously traveling in the zone to be cleaned.

The robot cleaner 200 according to the embodiment of the present invention is configured to be placed on a floor and clean the floor while moving on a floor surface. Therefore, hereinafter, an upward/downward direction is defined based on a state in which the robot cleaner 200 is placed on the floor.

Further, based on a pair of wheels 260, a side at which an auxiliary wheel 270 to be described below is defined as a front side, and a side at which the rotary cleaning part 240 to be described below is disposed is defined as a rear side.

Among the portions described in the embodiment of the present invention, a 'lowermost portion' may be a portion positioned at a lowest position or a portion closest to the floor when the robot cleaner 200 according to the embodiment of the present invention is placed on the floor and used.

The robot cleaner 200 according to the embodiment of the present invention includes a body 210, the dust bin 220, a water container 230, the rotary cleaning part 240, an agitator 250, the wheels 260, the auxiliary wheel 270, and a charging terminal 280.

The body 210 may define an overall external shape of the robot cleaner 200. Components constituting the robot cleaner 200 may be coupled to the body 210, and some of the components constituting the robot cleaner 200 may be accommodated in the body 210.

Specifically, the components of the robot cleaner 200 may be provided in a space in the body 210. For example, a battery and at least one motor may be accommodated in the space in the body 210.

In the embodiment of the present invention, the body 210 has a shape in which a width (or diameter) in a horizontal direction is larger than a height in an upward/downward direction. The body 210 may provide an advantageous structure that assists the robot cleaner 200 in having a stable structure and allows the robot cleaner 200 to avoid an obstacle while moving traveling.

The body 210 may have various shapes such as a circular shape, an elliptical shape, or a quadrangular shape when viewed from above or below.

The body 210 may be divided into a lower body and an upper body, and the lower body and the upper body may be coupled to define the space in the body 210.

The lower body may be coupled to the upper body to define the space capable of accommodating therein the battery, at least one sensor, and at least one motor.

A suction part 211 into which air is introduced may be formed in the lower body, and a hole configured to accommodate the pair of wheels 260 may be formed in the lower body.

The suction part 211 may be a passageway into which dust on the floor surface is introduced. Further, the suction part 211 may communicate with a suction flow path (not illustrated) formed in the body 210, and the suction flow path may communicate with an internal space of the dust bin 220.

Meanwhile, the lower body may be further provided with an air discharge flow path. One side of the air discharge flow path may communicate with the internal space of the dust bin 220, and the other side of the air discharge flow path may communicate with an air discharge outlet. In this case, a filter may be disposed in the air discharge outlet.

With this configuration, the air introduced through the suction part 211 may flow into the dust bin 220 through the suction flow path and be discharged to the air discharge outlet through the air discharge flow path.

The agitator 250 to be described below may be rotatably accommodated in the suction part 211. With this configuration, dust present around the suction part 211 may be guided into the suction part 211 by a rotation of the agitator 250, thereby improving efficiency in sucking dust.

The upper body may define an external appearance of an upper side of the robot cleaner 200. Although not illustrated, a display may be provided on the upper body.

The robot cleaner 200 of the present invention may include a bumper. The bumper is coupled along a rim of the body 210 and configured to move relative to the body 210.

The bumper may be coupled along a part of the rim of the body 210 or coupled along the entire rim of the body 210. At least one elastic member (not illustrated) may be provided between the bumper and the body 210. With this configuration, when the bumper comes into contact with an obstacle or the like and relatively moves toward a center of the body 210, the bumper may be returned to an original position by a restoring force of the elastic member (not illustrated), and the elastic member (not illustrated) may absorb or disperse impact applied to the bumper to prevent and reduce the transmission of the impact to the body 210.

The dust bin 220 may be provided to suck outside dust and air and store dust.

The dust bin 220 may store dust that passes through the suction flow path and is introduced. The dust bin 220 may have a dust inlet port configured to communicate with the suction flow path, the internal space capable of storing dust, and an air discharge port through which air may be discharged.

The dust bin 220 may be provided in the body 210. In this case, the dust bin 220 may be fixedly coupled to the body 210. Of course, according to the embodiment, the dust bin 220 may be separably provided.

Meanwhile, in the present invention, a dust discharge flow path may be formed in the dust bin 220. The dust discharge flow path may allow the internal space of the dust bin 220 and an external space of the robot cleaner 200 to communicate with each other. With this configuration, in case that the robot cleaner station 100 collects dust, the dust in the dust bin 220 may be removed.

Meanwhile, a dust discharge port 221, which communicates with the dust discharge flow path, may be formed in the dust bin 220 according to the embodiment of the present invention. For example, the dust discharge port 221 may be formed at one side of a rear side of an outer surface (or outer peripheral surface) of the body 210. In another example, the dust discharge port 221 may be formed in an outer surface of the dust bin 220.

In addition, the robot cleaner 200 according to the embodiment of the present invention may have a dust bin door 222 configured to selectively open or close the dust discharge port 221. Specifically, the dust bin door 222 may be coupled to the body 210 and disposed at a position at which the dust bin door 222 may block the dust discharge port 221. For example, the dust bin door 222 may be made of rubber or resin and configured to be flipped, and one side of the dust bin door 222 may be fixedly coupled to the body 210.

With this configuration, when a dust collection motor 145 of the robot cleaner station 100 to be described below operates, the dust bin door 222 is elastically deformed by driving power of the dust collection motor 145, and the dust discharge port 221 is opened, such that the dust in the dust bin 220 may be collected by a dust collection part 140 of the robot cleaner station 100.

The water container 230 may be provided in the form of a container having an internal space to store therein a liquid such as water. The water container 230 may be disposed in the body 210 and fixedly coupled to the body 210 or detachably coupled to the body 210.

The water container 230 include a supply part 231 and a nozzle (not illustrated). The supply part 231 may be configured to supply a liquid such as water from the outside. For example, the supply part 231 may have an injection port formed at the other side of the rear side of the outer surface (or outer peripheral surface) of the body 210, and the supply part 231 may be connected to a stomope space in the water container 230 through a water supply hose.

In this case, with a relationship with the dust discharge port 221, the supply part 231 may be disposed at a side opposite to the robot cleaner 200 in the leftward/rightward direction. For example, in case that the dust discharge port 221 is disposed at a rear left side of the body 210, the supply part 231 may be disposed at a rear right side of the body 210.

With the above-mentioned configuration, in the state in which the robot cleaner 200 is coupled to a robot cleaner station 100, the robot cleaner station 100 may perform both the dust collection and the water injection.

Meanwhile, the nozzle (not illustrated) is provided in the form of a tube or pipe and connected to the water container 230 so that the liquid in the water container 230 may flow through the interior of the nozzle. The nozzle (not illustrated) may be disposed such that one side thereof is connected to the water container 230, and the other end thereof is positioned above a pair of rotary plates 241 or positioned on each of the pair of rotary plates 241. Therefore, the liquid in the water container 230 may be supplied to the pair of mops 242.

That is, the nozzle (not illustrated) may be provided in a shape in which two tube portions are diverged from a single tube portion. In this case, an end of one diverged tube portion may be positioned above a left mop, and an end of the other diverged tube portion may be positioned above a right mop.

Meanwhile, although not illustrated, the water container 230 is equipped with a pump, and the pump may allow the water in the water container 230 to flow to the nozzle (not illustrated). Therefore, when the pump of the water container 230 operates, the liquid stored in the water container 230 may be discharged to the rotary cleaning part 240 through the nozzle (not illustrated).

The rotary cleaning part 240 includes the rotary plates 241 and the mops 242.

The rotary plates 241 may be provided as the pair of rotary plates 241 including a left rotary plate and a right rotary plate, and the mops 242 may be provided as the pair of mops 242 including the left mop and the right mop.

The rotary plate 241 may be rotatably disposed on a bottom surface of the body 210, and the mop 242 may be coupled to a lower side of the rotary plate 241.

The rotary plate 241 has a predetermined area and is provided in the form of a flat plate, a flat frame, or the like. The rotary plate 241 is laid approximately horizontally. Therefore, the rotary plate 241 has a shape in which a width (or diameter) thereof in the horizontal direction is sufficiently larger than a height thereof in the upward/downward direction. The rotary plate 241 coupled to the body 210 may be parallel to the floor surface or inclined with respect to the floor surface. The rotary plate 241 may have a circular plate-like shape, the bottom surface of the rotary plate 241 may have an approximately circular shape, and the rotary plate 241 may have a rotationally symmetrical shape as a whole.

The pair of rotary plates 241 may be vertically symmetric.

The mop 242 may be coupled to the lower side of the rotary plate 241 so as to face the bottom surface.

The mop 242 is configured such that a bottom surface of the mop 242 facing the floor has a predetermined area. The mop 242 has a flat shape. The mop 242 is configured such that a width (or diameter) thereof in the horizontal direction is sufficiently larger than a height thereof in the upward/downward direction. When the mop 242 is coupled to the body 210, the bottom surface of the mop 242 may be parallel to the floor or inclined with respect to the floor.

The bottom surface of the mop 242 may have an approximately circular shape, and the mop 242 may have a rotationally symmetrical shape as a whole. In addition, the mop 242 may be attached to or detached from the bottom surface of the rotary plate 241. The mop 242 may be coupled to the rotary plate 241 and rotate together with the rotary plate 241.

Meanwhile, although not illustrated, the rotary cleaning part 240 may be equipped with a drive part configured to apply a rotational force to the rotary plate 241. For example, the drive part may be provided with a motor and at least one gear. Therefore, when the drive part operates, the rotary plates 241 and the mops 242 may rotate to wipe and clean the floor surface.

The agitator 250 may have a plurality of brushes configured to be rotatable and guide outside dust and air into the dust bin 220. In this case, the agitator 250 may be provided with at least one gear.

Meanwhile, the agitator 250 according to the present embodiment may be equipped with a separate agitator motor (not illustrated) to receive rotational power. According to the embodiment, the agitator 250 may also receive rotational power from a driving motor or receive rotational power from the drive part of the rotary cleaning part 240.

The wheels 260 may be provided on the bottom surface of the body 210 and connected to the drive part (not illustrated). In this case, the drive part (not illustrated) may be coupled to the body 210.

The wheels 260 may be provided on the body 210 and roll on the floor.

The wheels 260 may include a first driving wheel and a second driving wheel. In this case, the first driving wheel may be identical to the second driving wheel, or the first driving wheel and the second driving wheel may be symmetric. For example, in case that the first driving wheel is positioned at the left side of the robot cleaner 200, the second driving wheel may be positioned at the right side of the robot cleaner 200. In this case, the first driving wheel and the second driving wheel may be symmetric vertically.

The drive part (not illustrated) may include driving motors and gears. In this case, the driving motors may be accommodated in the body 210 and provide power to the wheels 260. The driving motors may include a first driving motor and a second driving motor.

The driving motor may be configured as an electric motor. The plurality of gears engage with one another and rotate. The plurality of gears connect the driving motors and the wheels 260 and transmit rotational power of the driving motors to the wheels 260. Therefore, the wheels 260 may rotate when rotary shafts of the driving motors rotate.

With this configuration, when the driving motors operate, the wheels 260 may rotate, and the body 210 may travel on the floor surface at a predetermined traveling speed.

The auxiliary wheel 270 may be provided on a lower surface of the body 210 and roll on the floor surface (cleaning target surface). The auxiliary wheel 270, together with the pair of wheels 260, may support the body 210 on the floor surface. With this configuration, the auxiliary wheel 270 may guide a motion of the robot cleaner 200 while minimizing friction between the robot cleaner 200 and the floor surface.

The suction motor (not illustrated) may generate a suction force capable of sucking outside dust and air through the suction part 211. For example, the suction motor (not illustrated) may be an electric motor. By the suction force generated by the suction motor (not illustrated), outside dust and air may be introduced into the suction part 211 and reach the dust bin 220 after passing through the suction flow path.

Although not illustrated, the battery is coupled to the body 210 and configured to supply electric power to other components constituting the robot cleaner 200. The battery may supply electric power to at least one motor provided in the robot cleaner 200. For example, the battery may supply electric power to the motors provided in the rotary cleaning part 240, the agitator 250, the wheel 260, and the suction motor (not illustrated).

In addition, the battery may supply electric power to a sensor part (not illustrated) and a controller 300.

The battery may be charged with external electric power. To this end, the charging terminal 280 for charging the battery may be provided at one side of the body 210. For example, the charging terminal 280 may be disposed at a rear side of the outer surface of the body 210. The charging terminal 280 may be supplied with electric power by coming into contact with an electric power supply terminal 123b of the robot cleaner station 100 when the robot cleaner 200 is coupled to the robot cleaner station 100.

### ROBOT CLEANER STATION

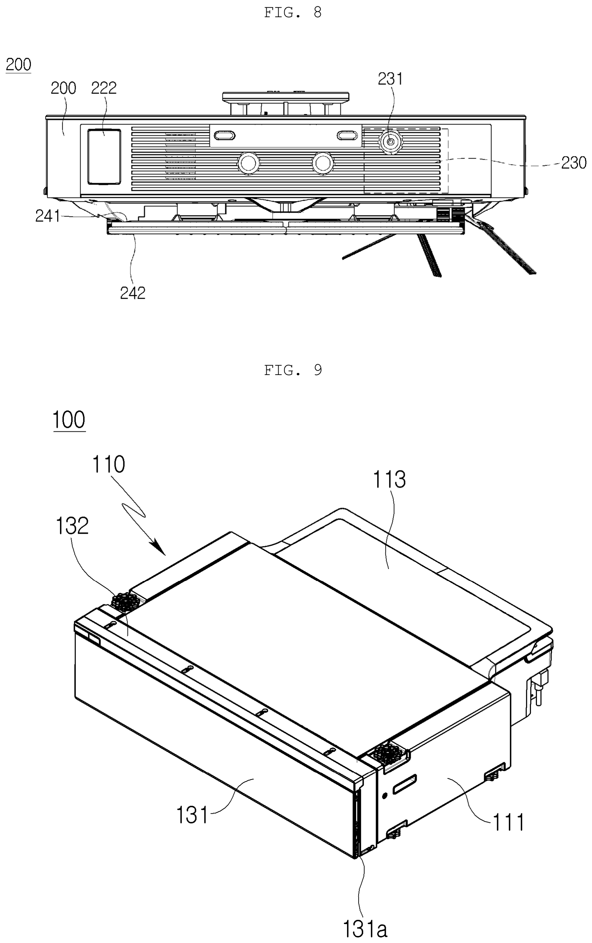

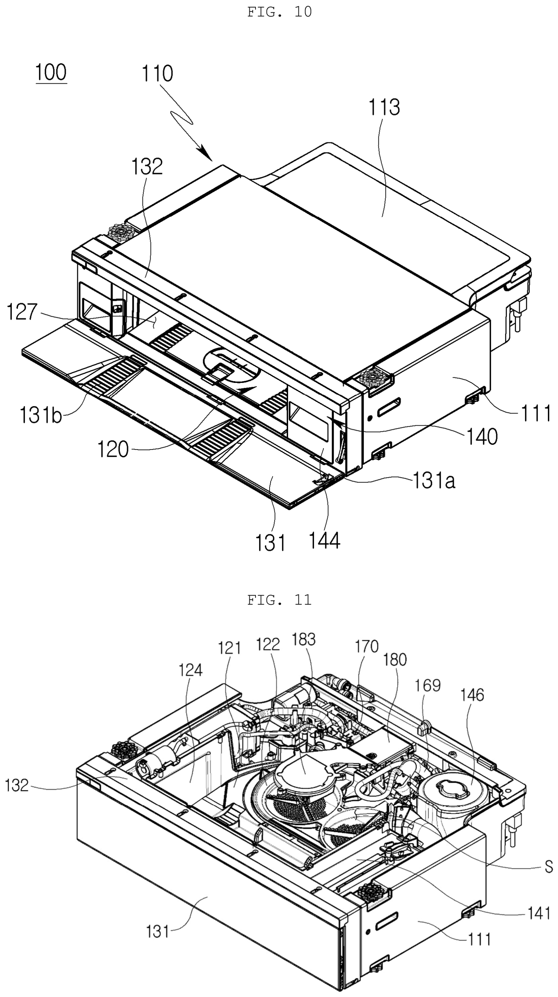

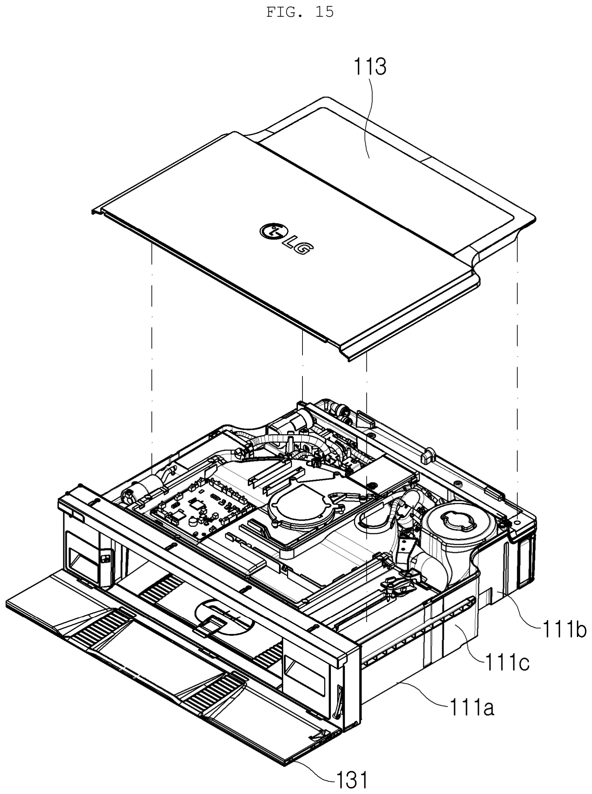

FIG. 9 is a perspective view for explaining a structure of the robot cleaner station according to the embodiment of the present invention. FIG. 10 is a perspective view for explaining a door part of the robot cleaner station according to the embodiment of the present invention. FIG. 11 is a perspective view for explaining an internal structure of the robot cleaner station according to the embodiment of the present invention. FIG. 15 is a view for explaining a state in which an upper cover is separated from the robot cleaner station according to the embodiment of the present invention.

The robot cleaner station 100 of the present invention will be described below with reference to FIGS. 3 to 11 and 15.

The robot cleaner 200 may be accommodated in the robot cleaner station 100. The robot cleaner 200 may be coupled to a seating part 120 of the robot cleaner station 100.

The robot cleaner station 100 may include a housing 110.

The housing 110 may define an external appearance of the robot cleaner station 100. For example, the housing 110 may be formed in a shape similar to a hexahedral shape including at least one outer wall surface.

The housing 110 may be formed with a space in which the seating part 120, a door part 130, the dust collection part 140, a mop washing part 160, a mop drying part 170, and an air discharge part 180 may be accommodated.

The housing 110 may be mounted below the kitchen cabinet 2. Specifically, the housing 110 may be installed in a mounting space formed between the lower plate 23 of the kitchen cabinet 2 and the floor of the kitchen.

The housing 110 includes a pair of outer walls 111 provided to face each other. The outer wall 111 may refer to a surface formed in a gravitational direction.

For example, the pair of outer walls 111 may be installed below the kitchen cabinet 2 at predetermined intervals. In this case, the housing 110 may further include a bottom surface 112 facing the floor of the kitchen, and the pair of outer walls 111 may be connected through the bottom surface 112.

Meanwhile, the housing 110 may further include an upper cover 113 facing the lower plate 23 of the kitchen cabinet 2, and the upper cover 113 may be separably coupled to upper ends of the pair of outer walls 111. Therefore, it is possible to prevent the constituent components of the robot cleaner 200 and the robot cleaner station 100 from being contaminated even in case that debris falls downward from the kitchen cabinet 2.

In addition, the upper cover 113 is formed in a shape in which two or more plates are superimposed. An inner cover 113b may refer to a plate disposed at a lowermost side among the plates. The inner cover 113b may cover an upper side of an accommodation space S. In addition, an outer cover 113a may refer to a plate disposed at an uppermost side of the upper cover 113. The outer cover 113a may face the lower plate 23 of the kitchen cabinet 2.

In addition, the housing 110 may further include a rear surface 111b facing the wall of the building. With this configuration, the constituent components of the robot cleaner station 100 may be accommodated in the housing 110 (between the pair of outer wall surfaces).

In addition, the robot cleaner 200 may be accommodated in the housing 110. The housing 110 may be disposed such that the pair of outer walls 111 have an interval larger than a maximum width of the robot cleaner 200 in the horizontal direction. With this configuration, the robot cleaner 200 may enter or exit the interior of the housing 110.

In this case, in the present embodiment, the robot cleaner 200 may enter or exit the front side of the robot cleaner station 100. In this case, the front side may refer to a direction in which a door 131 is provided based on the interior of the robot cleaner station 100.

In addition, the rear side may refer to a direction opposite to the front side based on the interior of the robot cleaner station 100. For example, the wall (not illustrated) of the building may be disposed rearward of the robot cleaner station 100. In addition, the left side may be referred to as a leftward side and the right side may be referred to as a rightward side when the robot cleaner station 100 is viewed from the outside.

That is, the outer walls 111 of the robot cleaner station 100 may be respectively disposed at the left side and the right side. An outer wall disposed at the left side may be referred to as a left surface 111c, and an outer wall disposed at the right side may be referred to as a right surface 111d. An inlet/outlet 127, through which the robot cleaner 200 enters or exits, may be formed at a front side of the housing 110. The rear surface 111b may be disposed at a side opposite to the inlet/outlet 127 in the housing 110.

Therefore, the upper side of the housing 110 may be covered by the kitchen cabinet 2, and the lower side of the housing 110 may be covered by the floor of the kitchen. In addition, the left and right surfaces of the housing 110 are kept covered by the outer walls, and the housing 110 is disposed below the kitchen cabinet 2. In this case, the lower portion of the kitchen cabinet 2, which excludes the robot cleaner station 100, is finished by a baseboard 26. As a result, only the front surface of the housing 110 may be exposed to the outside.

Therefore, it is possible to minimize a degree to which the robot cleaner station 100 and the robot cleaner 200 are exposed to the outside.

With this configuration, the robot cleaner station 100 of the present invention may provide an aesthetic appearance to a user in terms of interior aesthetics.

Meanwhile, the housing 110 may have a space through which a water supply hose connected to the water supply pipe passes, a space through which a water drain hose, through which wastewater produced after the process of washing the mop 242 is discharged, passes, and a space through which a hose, through which moisture produced during the process of drying the mop 242 is discharged, passes. For example, a space through which the hoses may pass may be formed in the outer wall 111 of the housing 110.

### LAYOUT

The robot cleaner station 100 according to the embodiment of the present invention is characterized by being installed in the lower space of the kitchen cabinet 2.

To this end, the robot cleaner station 100 according to the embodiment of the present invention is characterized by being disposed in the horizontal direction in accordance with a space formed between the lower plate 23 of the kitchen cabinet 2 and the floor of the kitchen.

Specifically, in the robot cleaner station 100 according to the embodiment of the present invention, the dust collection part 140 and/or the mop washing part 160 may be disposed on a lateral surface of an inlet/outlet 127.

In this case, in case that both the dust collection part 140 and the mop washing part 160 are provided, the seating part 120 may be disposed between the dust collection part 140 and the mop washing part 160.

For example, the inlet/outlet 127 and the door 131 may be disposed at the front side of the robot cleaner station 100. Further, the seating part 120 to which the robot cleaner 200 is coupled may be disposed rearward of the inlet/outlet 127. In this case, the dust collection part 140 may be disposed rearward by a predetermined length from a front end of the robot cleaner station 100. In addition, the mop washing part 160 may be disposed rearward by a predetermined length from the front end of the robot cleaner station 100.

Therefore, when the robot cleaner station 100 is viewed from a front outer side of the robot cleaner station 100, a front end of the dust collection part 140 and/or a front end of the mop washing part 160 may be disposed at left and right sides of the inlet/outlet 127.

In this case, a dust bag drawer 144 of the dust collection part 140 may be configured to be withdrawn forward from the housing 110. In addition, a detergent container 163 of the mop washing part 160 may be configured to be withdrawn forward from the housing.

Meanwhile, a rear end of a dust collection part housing 141 and a rear end of the detergent container 163 may be disposed to be spaced apart from a rear end of the housing 110 at a predetermined interval. Further, the dust collection motor 145 may be disposed between the rear end of the dust collection part housing 141 and the rear end of the housing 110. With this configuration, it is possible to minimize an entire space occupied by the seating part 120, the dust collection part housing 141, and the dust collection motor 145 within a limited space.

In addition, a flow path, through which the washing water for washing the mop 242 may flow, may be at least partially disposed between the rear end of the housing 110 and the rear end of the detergent container 163. With this configuration, a route along which the washing water is introduced from the water supply pipe may have a shortest distance. In addition, it is possible to minimize an entire space occupied by the seating part 120, the detergent container 163, and the flow path, through which the washing water flows, within a limited space.

Meanwhile, in the robot cleaner station 100, the mop drying part 170 may be disposed rearward of the seating part 120. In this case, the mop drying part 170 may be disposed between a rear end of the seating part 120 and the rear end of the housing 110.

Therefore, in the robot cleaner station 100 according to the embodiment of the present invention, the dust collection part 140 and the mop washing part 160 may be disposed on the left and right lateral surfaces based on the seating part 120, and the mop drying part 170 may be disposed at the rear side.

That is, in the robot cleaner station 100 according to the embodiment of the present invention, all the dust collection part 140, the mop washing part 160, and the mop drying part 170 are disposed within a predetermined distance range from an outer periphery of the seating part 120.

This provides an effect of shortening a distance between the dust bin 220 of the robot cleaner 200 and the dust collection part 140, thereby minimizing a flow path loss. In addition, it is possible to restrict a range in which the washing water is present and wastewater produced after the washing process is present by minimizing a distance between the mop 242 of the robot cleaner 200 and the mop washing part 160 and a distance between the mop 242 of the robot cleaner 200 and the mop drying part 170.

In addition, with the above-mentioned arrangement, all the constituent components of the robot cleaner station 100 of the present invention may be disposed within a limited height.

As a result, in the robot cleaner station 100 according to the embodiment of the present invention, the dust collection part 140, the mop washing part 160, and the mop drying part 170 may be disposed on the three surfaces that surround the seating part 120, except for the front surface into which the robot cleaner 200 is introduced. With the above-mentioned arrangement, even in a situation in which a height in the vertical direction is restricted, the minimum horizontal space may be used not only to charge the robot cleaner 200, but also to collect dust from the robot cleaner 200, wash the mop 242, and dry the mop 242.

### SEATING PART

As illustrated in FIGS. 10 to 11, the robot cleaner station 100 may include the seating part 120.

The robot cleaner 200 and the robot cleaner station 100 may be connected through the seating part 120 in a physical, electrical, and/or flow path manner.

The seating part 120 may be disposed in the housing 110.

The inlet/outlet 127 into which the robot cleaner 200 is introduced may be formed at a front side of the seating part 120. The inlet/outlet 127 may refer to a space formed in the front surface of the robot cleaner station 100.

The inlet/outlet 127 may be sized to allow the robot cleaner 200 to pass therethrough. That is, a height of the inlet/outlet 127 is larger than a height of the robot cleaner 200. In this case, the inlet/outlet 127 may refer to a space formed upward in the vertical direction from a front end of the base 121 to be described below. Alternatively, the inlet/outlet 127 may refer to a hole formed in a door frame 132 to be described below so that the robot cleaner 200 passes through the hole.

At least one of the dust collection part 140 and the mop washing part 160 may be disposed at the left and right sides of the inlet/outlet 127. Therefore, the left and right ends of the inlet/outlet 127 may define a boundary with the dust collection part 140 and the mop washing part 160.

In this case, the inlet/outlet 127 may be opened or closed by the door 131.

The seating part 120 may include an accommodation space S, a base 121, a coupling wall 123, and inner walls 124.

The robot cleaner 200 may be accommodated in the accommodation space S of the seating part 120. For example, the accommodation space S may refer to a space surrounded by the base 121, the coupling wall 123, and the inner walls 124. In another example, the accommodation space S may refer to a space surrounded by the base 121, a washing plate 122, the coupling wall 123, and the inner walls 124. In still another example, the accommodation space S may refer to a space in which the robot cleaner 200 is positioned in a state in which the robot cleaner 200 is coupled to the electric power supply terminal 123b or a space in which the robot cleaner 200 is positioned in a state in which the dust bin 220 of the robot cleaner 200 communicates with a dust passage hole 123a. The accommodation space S may refer to a space formed between the upper cover 113 and the base 121 disposed at the lower side of the robot cleaner.

The base 121 may be configured to support the robot cleaner 200 in case that the robot cleaner 200 is coupled to the robot cleaner station 100. The wheel 260 of the robot cleaner 200 may come into contact with an upper surface of the base 121. In addition, the auxiliary wheel 270 of the robot cleaner 200 may come into contact with the upper surface of the base 121.

The base 121 may include a base main body 121a, an inclined portion 121b, wheel coupling portions 121c, an agitator accommodation portion 121d, and a washing tub 128.

The base main body 121a may define an overall external shape of the base 121. The inclined portion 121b, the wheel coupling portions 121c, the agitator accommodation portion 121d, and the washing tub 128 may be disposed on the base main body 121a.

The base main body 121a may have a shape in which a length and width thereof in the horizontal direction are larger than a height thereof in the upward/downward direction. With this structure, the robot cleaner station 100 may be stably supported on the floor surface.

A return flow path may be provided in the base main body 121a. Therefore, air discharged from the dust collection motor 145 may flow in a return flow path 125a formed in the base main body 121a and be discharged to an air return port 125b.

The inclined portion 121b may be disposed in an inlet of the base main body 121a on which the robot cleaner 200 climbs.

The inclined portion 121b may be inclined upward and forward in the direction in which the robot cleaner 200 enters. More specifically, a front end of the inclined portion 121b may be connected to the ground surface without a height difference and inclined upward and rearward. That is, the inclined portion 121b may be formed to be gradually raised from the ground surface when the robot cleaner 200 enters. Therefore, the robot cleaner 200 may easily climb from the ground surface to the robot cleaner station 100.

The wheels 260 of the robot cleaner 200 raised along the wheel guide portions 121ba may be seated on the wheel coupling portions 121c. When the wheel 260 of the robot cleaner 200 is seated on the wheel coupling portion 121c, the robot cleaner 200 and the robot cleaner station 100 may be physically coupled. The surface of the wheel coupling portion 121c may be formed to correspond to the surface of the wheel 260 so that the robot cleaner 200 may be stably stopped. The wheel coupling portion 121c may extend from an upper end of the wheel guide portion 121ba. The wheel coupling portion 121c may be connected to the wheel guide portion 121ba without a level difference. Therefore, the robot cleaner 200 may easily move to the wheel coupling portion 121c while passing over the inclined portion 121b.

The wheel coupling portions 121c may be disposed at stop positions of the left and right wheels 260 of the robot cleaner 200 so that the robot cleaner 200 is stopped at an exact position. In this case, the stop position of the wheel 260 refers to a position determined so that the robot cleaner 200 is stopped to be coupled to the electric power supply terminal 123b and/or a position determined so that the dust bin 220 of the robot cleaner 200 is stopped to communicate with the dust passage hole 123a.

A shape of the wheel coupling portion 121c may be a shape, i.e., an arcuate shape corresponding to a shape of the wheel 260 of the robot cleaner 200. With this configuration, the robot cleaner 200 may move along the wheel guide portion 121ba and be stopped at the same time when the wheel 260 is inserted into the wheel coupling portion 121c, and the wheel 260 may be stably seated on the wheel coupling portion 121c having an arcuate shape.

At least a part of the agitator 250 of the robot cleaner 200 may be accommodated in the agitator accommodation portion 121d.

The agitator accommodation portion 121d may be formed between the wheel coupling portions 121c. The agitator accommodation portion 121d may be formed in a shape corresponding to the agitator 250 of the robot cleaner 200. The agitator accommodation portion 121d may be formed in a rectangular parallelepiped shape opened at an upper side thereof. A lower surface of the agitator accommodation portion 121d may be sealed by a bottom surface of the base main body 121a or a bottom surface of the housing 110. Therefore, the agitator 250 of the robot cleaner 200, which is moved upward along the inclined portion 121b, may be seated in the agitator accommodation portion 121d. In this case, a depth of the agitator accommodation portion 121d may be smaller than a depth of the wheel coupling portion 121c.

The agitator accommodation portion 121d may be recessed in the base main body 121a. Therefore, the agitator accommodation portion 121d may provide a space that accommodates a lower end of the agitator 250 in the state in which the wheels 260 of the robot cleaner 200 are seated on the wheel coupling portions 121c.

The air return port 125b may be formed in the agitator accommodation portion 121d. The air return port 125b may be formed in a lateral surface of the agitator accommodation portion 121d. The air return port 125b may connect the agitator accommodation portion 121d and the dust collection motor 145 through the return flow path. The agitator accommodation portion 121d and the return flow path may communicate with each other through the air return port 125b. Therefore, the air discharged from the dust collection motor 145 may pass through the air return port 125b and be discharged to the agitator accommodation portion 121d.

The agitator accommodation portion 121d may guide air, which is discharged through the air return port 125b, to the suction part 211 of the robot cleaner 200.

Meanwhile, a drawer 190 may be provided on the base 121 and configured to be withdrawable from the housing 110. In this case, the base 121 may be withdrawn while passing through the inlet/outlet 127 along a space between the inner walls 124.

In order to facilitate the withdrawal, the base 121 may be formed with a base handle 121e. The base handle 121e may be formed between the agitator accommodation portion 121d and the auxiliary wheel guide portion 121bb. In addition, the base handle 121e may be formed between the pair of wheel guide portions 121ba.

The base handle 121e may be formed in a shape obtained by recessing the base main body 121a from a rear side thereof toward a front lower side thereof. For example, the base handle 121e may be formed in the shape of an elliptical groove, a cover may be provided at a front side of the base handle 121e, and a rear side of the base handle 121e is open.

With this configuration, the user may easily withdraw the base 121 by holding and pulling the handle 121e.

The coupling wall 123 is a component in which the dust passage hole 123a, the electric power supply terminal 123b, and a water supply nozzle 123c of the robot cleaner station 100 are disposed. The coupling wall 123 may spatially distinguish the accommodation space S from components of the robot cleaner station 100. The coupling wall 123 may extend in the vertical direction from a rear side of the base 121. The coupling wall 123 may be formed to correspond to a shape of the robot cleaner 200. For example, in case that the body 210 of the robot cleaner 200 has a cylindrical shape, the coupling wall 123 may be formed in a circular arc shape having a predetermined radius. With this configuration, it is possible to increase an area that may surround an outer periphery of the robot cleaner 200 and face the outer surface of the robot cleaner 200. In addition, the robot cleaner 200 may be stably supported.

The seating part 120 may be formed with the dust passage hole 123a through which air existing outside the housing 110 may be introduced into the housing 110. Specifically, the dust passage hole 123a may be formed in the coupling wall 123 so that outside air may be introduced into the housing 110. In this case, the dust passage hole 123a may be disposed at a rear side of the dust collection part housing 141 to be described below.

The dust passage hole 123a may communicate with the dust bin 220 of the robot cleaner 200. The dust passage hole 123a may communicate with the dust discharge port 221 of the dust bin 220 of the robot cleaner 200. The dust passage hole 123a may be formed in the form of a hole corresponding to a shape of the dust bin 220 so that the dust in the dust bin 220 may be introduced into the dust collection part 140.

The dust passage hole 123a may be formed to communicate with dust collection flow paths 147 and 148. The air sucked into the dust passage hole 123a may flow along the dust collection flow paths 147 and 148 and then be discharged through the air return port 125b.

The robot cleaner station 100 may include an electric power supply module configured to supply electric power to the robot cleaner 200. The electric power supply module may include an electric power supply module housing and the electric power supply terminal 123b, and a circuit board and an element for supplying electric power may be mounted in the electric power supply module housing. Further, the electric power supply terminal 123b may be disposed forward in the electric power supply module housing and disposed on the coupling wall 123 so as to be exposed.

The electric power supply terminal 123b may supply electric power to the robot cleaner 200 coupled to the seating part 120. The electric power supply terminal 123b may come into contact with and be electrically connected to the charging terminal of the robot cleaner 200. The electric power supply terminal 123b may be disposed on the seating part 120. Specifically, the electric power supply terminal 123b may be disposed on the coupling wall 123. The electric power supply terminal 123b may be electrically connected to the robot cleaner 200 coupled to the coupling wall 123. The electric power supply terminal 123b may supply electric power to the battery of the robot cleaner 200 coupled to the coupling wall 123.

The robot cleaner station 100 may further include the water supply nozzle 123c.

The water supply nozzle 123c may be connected to the supply part 231 of the water container 230 of the robot cleaner 200. Specifically, the water supply nozzle 123c may be connected to the injection port of the water container 230. The water supply nozzle 123c may supply water, which is supplied from the water supply pipe of the kitchen cabinet 2, to a stomope space in the water container 230 of the robot cleaner 200.

In addition, the robot cleaner station 100 may further include a coupling guide 123d. The coupling guide 123d may be coupled to a coupling groove formed in the robot cleaner 200 in the state in which the robot cleaner 200 is seated on the seating part 120. Therefore, the coupling guide 123d may guide the robot cleaner 200 so that the robot cleaner 200 is accurately coupled at an exact position. The coupling guide 123d may be disposed between a pair of outside air discharge parts 173.

In addition, the coupling guide 123d may include a sensor configured to detect a coupled state. Therefore, it is possible to detect whether the robot cleaner 200 is accurately seated.

The inner walls 124 is configured to spatially distinguish the accommodation space S of the seating part 120 from the components of the robot cleaner station 100. The inner walls 124 may be provided as a pair of inner walls 124 disposed at the left and right sides of the base 121. The inner walls 124 may be connected to two opposite ends of the coupling wall 123. The inner walls 124 may extend from the left and right sides of the base 121 in a direction intersecting the base 121. Specifically, the inner walls 124 may extend from the left and right sides of the base 121 in the vertical direction.

Meanwhile, various types of components, such as dust collection flow paths 147 and 148, the dust collection part 140, the dust collection motor 145, a detergent container 163, and a wastewater container 166 may be disposed outside the inner wall 124. Specifically, the dust collection part 140, the detergent container 163, and the wastewater container 166 may be disposed in a space between the inner wall 124 and the outer wall 111 of the housing 110.

The dust collection part 140 and the detergent container 163 may be separated in a sliding manner from the space between the inner wall 124 and the outer wall 111 of the housing 110. Widths of the dust collection part 140 and the detergent container 163 in the leftward/rightward direction may be smaller than a distance between the inner wall 124 and the outer wall 111 of the housing 110.

The washing plate 122 may be configured to wash the mop of the robot cleaner 200, and the washing plate 122 may be seated in the washing tub 128 of the base 121. In addition, the washing plate 122 may come into contact with the mop 242 in the state in which the robot cleaner 200 is seated.

The washing plate 122 may be a plate inclined downward toward a central portion thereof as a whole.

Specifically, the washing plate 122 includes a flow guide surface 122c formed as a curved surface. Further, one or more passing holes 122b through which the fluid may pass may be formed in the flow guide surface 122c. In addition, washing protrusions 122a may protrude from the flow guide surface 122c.

In this case, the washing protrusions 122a may be provided as a pair of washing protrusions 122a symmetrically formed on the flow guide surface 122c. Specifically, the pair of washing protrusions 122a may be disposed vertically below the pair of mops 242 of the robot cleaner 200 and disposed to face the pair of mops 242. The pair of washing protrusions 122a may be disposed to at least partially come into contact with the pair of mops 242.

Further, the passing holes 122b may be provided as a plurality of passing holes 122b formed in the flow guide surface 122c and formed between the pair of washing protrusions 122a. For example, the plurality of passing holes 122b may be formed while including positions on the flow guide surface 122c that have the lowest heights from the ground surface (the floor of the kitchen), and the plurality of passing holes 122b may be formed between the pair of washing protrusions 122a. Therefore, the fluid discharged between the pair of washing protrusions 122a may flow while being guided to the passing holes 122b.

Meanwhile, a height of the flow guide surface 122c from the floor of the kitchen may increase rearward from the position at which the passing holes 122b are formed. That is, the height of the flow guide surface 122c from the floor of the kitchen may increase as the distance from the outside air discharge parts 173 to be described below decreases.

With this configuration, the washing water and/or air may flow along the flow guide surface 122c and enter a space formed between the washing plate 122 and the washing tub 128 through the passing holes 122b.

When the washing water is supplied to the washing plate 122 and the mop 242 rotates, the mop 242 may be washed by friction with the washing protrusions 122a provided in a stationary state.

Meanwhile, at least a part of the washing plate 122 may be disposed above a flow path forming portion 128c to be described below. That is, the washing plate 122 may further include a return flow path cover portion 122d protruding upward from the flow guide surface 122c and coupled to an upper side of the flow path forming portion 128c.

The washing plate 122 of the present embodiment may be formed in a shape corresponding to a shape of the flow path forming portion 128c. For example, a front left portion of the washing plate 122 may protrude upward from the flow guide surface 122c and cover the flow path forming portion 128c disposed therebelow.

With this configuration, the washing plate 122 and the washing tub 128 may be accurately coupled while simultaneously providing a sufficient space in which the return flow path 125a may be formed.

The washing tub 128 is a component in which the washing plate 122 is seated. The washing tub 128 may be disposed rearward of the base main body 121a. The washing tub 128 may be disposed below the washing plate 122 and detachably coupled to the washing plate 122. The washing tub 128 may be formed to correspond to the washing plate 122 so as to be fitted with the washing plate 122. The liquid passing through the washing plate 122 may be introduced into the washing tub 128.

The washing tub 128 may include a washing tub base surface 128a along which the fluid having passed through the washing plate 122 flows, and a washing tub wall 128b extending and protruding in the vertical direction from an outer periphery of the washing tub base surface 128a. In this case, a height of the washing tub base surface 128a from the ground surface (the floor of the kitchen) may decrease toward the rear side of the robot cleaner station 100. Therefore, the fluid having passed through the washing plate 122 may be collected at a rear side of the washing tub 128 and discharged to the outside through a wastewater inlet port 164c to be described below.

In this case, a wastewater pipe connection port 128d may be formed in the washing tub wall 128b so as to be coupled to the wastewater inlet port 164c.

Meanwhile, the washing tub 128 may be formed with the flow path forming portion 128c. The flow path forming portion 128c may protrude upward from the washing tub base surface 128a and form the return flow path 125a therebelow. Specifically, at least a part of the return flow path 125a may be formed between a lower surface of the base 121 and the flow path forming portion 128c.

Meanwhile, the washing tub 128 according to the present invention may be configured to be withdrawable from the mop washing part 160. That is, the washing tub 128, together with the base 121, may be withdrawn from the housing 110. In addition, the washing plate 122 may also be withdrawn from the mop washing part 160.

### DOOR PART

The door part 130 may be provided to cover an entire front end of the housing 110. The door 131 may cover the dust bag drawer 144 and the detergent container 163 so that the dust bag drawer 144 and the detergent container 163 are not exposed to the outside.

The door 131 may form a front external appearance of the robot cleaner station 100 in a state in which the inlet/outlet 127 is closed. For example, the door 131 may be formed in a shape similar to a rectangular flat plate-like shape. A length of the door 131 in the leftward/rightward direction may be equal to or longer than a length of the housing 110 in the leftward/rightward direction. With this configuration, the dust bag drawer 144 and the detergent container 163 may be protected from the outside, and a neat external appearance of the robot cleaner station 100 may be implemented.

The door frame 132 may be disposed at a front side of the housing 110. The door 131 may be openably and closably coupled to the door frame 132. In addition, the door frame 132 may be formed with a dust bag withdrawal port 132a to which the dust bag drawer 144 and the inlet/outlet 127, through which the robot cleaner 200 may enter or exit, are coupled to be withdrawable, and a detergent container insertion port 132b to which the detergent container 163 is coupled to be withdrawable.

The door frame 132 may define a front external appearance of the robot cleaner station 100 in the state in which the door 131 is open.

At least one surface of the dust bag drawer 144, which is coupled to the door frame 132, and at least one surface of the detergent container 163 may be provided to be exposed to the outside in the state in which the door 131 opens the inlet/outlet 127. When the door 131 opens the inlet/outlet 127, a front surface of the dust bag drawer 144 and a front surface of the detergent container 163 are exposed to the outside, and a handle 144d of the dust bag drawer 144 and a handle 163b of the detergent container 163 are exposed to the outside. With this configuration, the dust bag drawer 144 and the detergent container 163 are easily withdrawn or retracted, and a neat external appearance may be provided.

A rotary shaft 131a of the door 131 may be disposed at a lower end of the door frame 132. When the inlet/outlet 127 is open, the door 131 may be disposed in parallel with the floor surface or inclined downward and forward, such that an end thereof may come into contact with the ground surface.

The door 131 may be provided with a hinge part and be rotatably connected to the door frame 132. The hinge part may be provided as a plurality of hinge parts disposed along the rotary shaft 131a and spaced apart from one another at different intervals.

In addition, auxiliary entry paths 131b may be provided on a surface of the door 131 that faces the housing 110 when the inlet/outlet 127 is closed. The auxiliary entry paths 131b may be provided to allow the robot cleaner 200 to stably travel to the seating part 120 or the inlet/outlet 127, and the auxiliary entry paths 131b may be provided to be inclined upward and rearward.

Specifically, the auxiliary entry paths 131b may be formed in the shape of grooves to allow the robot cleaner 200 to stably travel inclinedly. The auxiliary entry paths 131b may be configured such that the grooves formed in the leftward/rightward direction are disposed to be spaced apart from one another at equal intervals in a forward/rearward direction. The auxiliary entry path 131b may be formed such that a width thereof decreases rearward in the leftward/rightward direction. Therefore, the wheel 260 of the robot cleaner 200 may be guided to the exact position while the leftward and rightward movements of the wheel 260 are restricted as the wheel 260 travels toward the seating part 120 or the inlet/outlet 127.

The auxiliary entry paths 131b guide the wheel 260 to the wheel guide portions 121ba disposed on the seating part 120. The auxiliary entry paths 131b may be provided as a pair of auxiliary entry paths 131b respectively disposed at positions connected to the pair of wheel guide portions 121ba.

Meanwhile, the door 131 may operate in accordance with whether the robot cleaner 200 approaches, whether the robot cleaner 200 begins to travel, or the like. Alternatively, the door 131 may operate in response to an input applied to a door manipulation part 133.

An entry sensor 135 may be installed on the door frame 132 and recognize the approach of the robot cleaner 200. The entry sensor 135 may be disposed at the front side of the housing 110 to recognize the approach of the robot cleaner 200. For example, the entry sensor 135 may be an IR sensor.

The entry sensor 135 may be installed at an upper side of a front surface of the door frame 132. Therefore, it is possible to maximize a detection range. In addition, the entry sensor 135 may be installed at a central portion of the inlet/outlet 127 based on the leftward/rightward direction. Therefore, an entry direction of the robot cleaner 200 may be guided in accordance with communication with the robot cleaner 200.

Meanwhile, the door 131 may be formed in a shape in which a portion facing the entry sensor 135 is cut out so that the entry sensor 135 may detect a front location even in the state in which the door 131 closes the inlet/outlet 127. Alternatively, the door 131 may be provided with a transparent window provided at a position that faces the entry sensor 135.

Meanwhile, the door 131 may be opened while a steam supply part 169 operates. The door 131 may be opened to supply outside air to the accommodation space S while the steam supply part 169 discharges water or wet vapor into the accommodation space S.

The door manipulation part 133 may be installed on the door frame 132, and the user may rotate the door 131 by manipulating the door manipulation part 133.

The door manipulation part 133 may be provided with at least one button disposed on the door frame 132 and configured to operate the door 131. The door manipulation part 133 may rotate the door 131 regardless of a position or state of the robot cleaner 200. The door manipulation part 133 may have one button configured to open or close the door 131 or have a button configured to open the door 131, and a button configured to close the door 131.

The door manipulation part 133 may be disposed in the door frame 132. Further, at least one button of the door manipulation part 133 may be disposed to be exposed to the outside.

In this case, the button may be disposed adjacent to the detergent container 163 based on the inlet/outlet. For example, the button may be disposed above the handle 163b of the detergent container 163.

Meanwhile, when the door 131 closes the inlet/outlet 127, the door 131 also covers the door manipulation part 133. In this case, external button parts 131c are provided on the door 131 so that the door manipulation part 133 may be manipulated even in the state in which the inlet/outlet 127 is closed. The external button parts 131c are configured to be equal in number to the buttons of the door manipulation part 133 and provided at positions facing the buttons. The external button part 131c may be made of an elastically deformable material and configured to press the button when an external force is applied.

The door 131 may be rotated by a door driving part 134. For example, the door driving part 134 may include a door driving motor and a driving gear part.

The door driving motor may be disposed in the housing 110 and disposed in an upper space of the detergent container 163. The door driving motor may be disposed between the detergent container 163 and the upper cover 113 of the housing 110. In addition, the door driving motor may be disposed at a front side of a space provided between the outer wall 111 and the seating part 120. That is, the door driving motor may be disposed adjacent to the door manipulation part 133.

Therefore, the spatial utilization and accessibility are improved, thereby providing convenience to the user.

The driving gear part is configured to connect the door driving motor and the door 131 and transmit power. The driving gear part rotates the door 131 by transmitting driving power from the door driving motor to the door 131.

### DUST COLLECTION PART

The dust collection part 140 may collect dust from the dust bin 220 of the robot cleaner 200. The dust collection part 140 may be disposed in the housing 110. The dust collection part 140 may be disposed outside the seating part 120. That is, the dust collection part 140 may be disposed between the housing 110 and the seating part 120. For example, the dust collection part 140 may be disposed at one side of the seating part 120 based on a leftward/rightward direction.

The dust collection part 140 may include the dust collection part housing 141, a filter 142, a dust bag 143, the dust bag drawer 144, the dust collection motor 145, a dust collection motor housing 146, a first dust collection flow path 147, and a second dust collection flow path 148.

The dust collection part housing 141 may form a space in which the filter 142, the dust bag 143, and the dust bag drawer 144 may be accommodated.

The dust bag drawer 144, which may be withdrawn, may be coupled in the dust collection part housing 141, and the dust bag 143 may be accommodated in the dust bag drawer 144. For example, the dust collection part housing 141 may be provided in the form of a rectangular tube opened at a front side thereof, and a rear internal space may communicate with the first dust collection flow path 147 and the second dust collection flow path 148.

Dust in the dust bin 220 may be introduced into the dust collection part housing 141.

One side of the inside of the dust collection part housing 141 may communicate with the first dust collection flow path 147, and the other side of the inside of the dust collection part housing 141 may communicate with the second dust collection flow path 148. In addition, when the dust bag 143 is coupled to the dust collection part housing 141, the dust bag 143 may be disposed in the dust collection part housing 141 and communicate with the first dust collection flow path 147.

The filter 142 may be disposed in the dust bag drawer 144. Specifically, the filter 142 may be disposed in the dust bag drawer 144 and withdrawn together with the dust bag drawer 144.

Meanwhile, the filter 142 may be disposed to be lower than the inlet port 144b based on the bottom surface of the dust bag drawer body 144a. The filter 142 may be disposed forward of the outlet port 144c of the dust bag drawer 144.

Specifically, the filter 142 may be detachably coupled to the lower surface of the dust bag drawer 144. In this case, the filter 142 may be disposed at one end (front end) of the flow path forming portion 144e based on the longitudinal direction. Therefore, the filter 142 may be disposed between the handle 144d and the flow path forming portion 144e. That is, the filter 142 may be disposed adjacent to a front surface of the dust bag drawer 144.

When the dust bag drawer 144 is withdrawn, the filter 142 may be withdrawn together with the dust bag drawer 144. That is, when the handle 144d is pulled, the filter 142 may be withdrawn together with the dust bag drawer 144. In this case, because the filter 142 is disposed immediately rearward of the handle 144d, the user may easily replace the filter 142 even in a state in which only a part of the dust bag drawer 144 is withdrawn.

Meanwhile, the filter 142 may be disposed to be spaced apart from two opposite surfaces of the dust bag drawer 144 based on the leftward/rightward direction (width direction). That is, a space may be formed between the filter 142 and two opposite surfaces of the dust bag drawer body 144a based on the leftward/rightward direction. In this case, the user's finger may enter the space.

With this configuration, the user may separate the filter 142 by a simple operation of inserting the finger into the space between the filter 142 and the dust bag drawer body 144a and pulling the filter 142.

The filter 142 may be disposed below the dust bag 143. In this case, the dust bag 143 may be coupled to be detachable from the dust bag drawer 144 in the vertical direction in a sliding manner.

Therefore, the dust bag 143 may be separated in the vertical direction from the dust bag drawer 144 in the state in which the dust bag drawer 144 is withdrawn. The filter 142 may be exposed to the outside when the dust bag 143 is separated.

Therefore, the user may identify a state of the filter 142 each time the user replaces the dust bag 143. The user may easily replace the filter 142 while replacing the dust bag 143.

The dust bag 143 may refer to a dust collection bag configured to collect dust sucked from the interior of the dust bin 220 of the robot cleaner 200 by the dust collection motor 145.

The dust bag 143 may be detachably coupled to the dust bag drawer 144. Therefore, the dust bag 143 may be separated from the dust bag drawer 144 and discarded, and a new dust bag 143 may be coupled to the dust bag drawer 144. That is, the dust bag 143 may be defined as a consumable component.

An inlet port of the dust bag 143 may be disposed to communicate with the inlet port 144b of the dust bag drawer 144. Therefore, when the dust collection motor 145 operates, air and dust in the dust bin 220 may be introduced into the dust bag 143 and captured.

When a suction force is generated by the dust collection motor 145, a volume of the dust bag 143 is increased, such that the dust may be accommodated in the dust bag 143. To this end, the dust bag 143 may be made of a material that transmits air but does not transmit debris such as dust. For example, the dust bag 143 may be made of a non-woven fabric material and have a hexahedral shape corresponding to a shape of the dust bag drawer 144 when the bag 143 has an increased volume.

The dust collection part 140 may further include a dust collection module. The dust collection module may provide a suction gas flow to the dust collection flow path.

Specifically, the dust collection part 140 may further include the dust collection motor 145 and the dust collection motor housing 146.

The dust collection motor 145 may generate a suction force in the dust collection flow paths 147 and 148. That is, the dust collection motor 145 may provide a suction force that sucks the dust in the dust bin 220 into the dust bag 143 disposed in the dust collection part housing 141.

The dust collection motor 145 may be disposed rearward of the dust collection part housing 141. Therefore, the dust collection motor 145 may provide a suction force capable of sucking dust in the dust bin 220 of the robot cleaner 200.

The dust collection motor 145 may generate the suction force by means of the rotation. For example, although not illustrated, the dust collection motor 145 may include a rotor and a stator that perform a relative rotation by receiving electric power, and the dust collection motor 145 may include an impeller configured to be rotated about a rotary shaft by the rotation of the rotor. Therefore, the suction force may be generated by the rotation of the impeller.

One side of the dust collection motor 145 may be connected to the second dust collection flow path 148, and the other side of the dust collection motor 145 may be connected to the return flow path 125a. In case that the dust collection motor 145 operates, the air flowing through the second dust collection flow path 148 may be introduced into the dust collection motor housing 146. In addition, the air introduced into the dust collection motor housing 146 may pass through the dust collection motor 145 and then flow through the return flow path 125a.

Meanwhile, in the present embodiment, a rotary shaft of the dust collection motor 145 may be disposed in the vertical direction. In this case, it is possible to minimize a horizontal space occupied by the dust collection motor 145.

Meanwhile, in case that the rotary shaft of the dust collection motor 145 is disposed in the vertical direction, a side at which the air is introduced into the dust collection motor 145 and a side at which the air is discharged from the dust collection motor 145 may be disposed at different heights. Therefore, a structure of the dust collection motor housing 146 may be formed.

The dust collection motor 145 may be accommodated in the dust collection motor housing 146. The dust collection motor housing 146 may be disposed rearward of the dust collection part housing 141. In addition, the dust collection motor housing 146 may be disposed rearward of the first dust collection flow path 147. In addition, the dust collection motor housing 146 may be disposed rearward of the second dust collection flow path 148.

That is, based on the forward/rearward direction of the robot cleaner station 100, the dust collection part housing 141 may be disposed at a foremost side, and the first dust collection flow path 147 and the second dust collection flow path 148 may be disposed rearward of the dust collection part housing 141. In addition, the dust passage hole 123a may be disposed rearward of the first dust collection flow path 147, and the dust collection motor housing 146 may be disposed rearward of the second dust collection flow path 148. In addition, the dust collection motor housing 146 may be disposed rearward of the dust passage hole 123a.

Therefore, the dust collection part 140 may be disposed in the forward/rearward direction of the robot cleaner station 100, as a whole, thereby reducing an overall height.

Meanwhile, the dust collection part 140 may further include the dust collection flow paths 147 and 148. The dust collection flow path may refer to a flow path through which the air sucked through the dust passage hole 123a flows to the dust collection motor 145 via the dust bag.

Specifically, the dust collection flow path may include the first dust collection flow path 147 configured to allow the dust bin 220 and an internal space of the dust collection part housing 141 to communicate with each other when the robot cleaner 200 is coupled to the robot cleaner station 100 and the dust passage hole 123a and the dust bin 220 of the robot cleaner 200 communicate with each other, and the second dust collection flow path 148 configured to allow the internal space of the dust collection part housing 141 and an internal space of the dust collection motor housing 146 to communicate with each other.

The first dust collection flow path 147 may connect the dust bin 220 of the robot cleaner 200 and the internal space of the dust collection part housing 141. The first dust collection flow path 147 may connect the dust passage hole 123a of the seating part 120 and the internal space of the dust collection part housing 141. The first dust collection flow path 147 may be formed in a direction intersecting the vertical direction. For example, the first dust collection flow path 147 may be formed to be close to the horizontal direction. The first dust collection flow path 147 may be a space formed rearward of the dust passage hole 123a. The first dust collection flow path 147 may be a flow path bent laterally from the dust passage hole 123a, and the dust and the air may flow through the first dust collection flow path 147. The dust in the dust bin 220 of the robot cleaner 200 may move to the internal space of the dust collection part housing 141 through the first dust collection flow path 147.

The second dust collection flow path 148 may connect the internal space of the dust collection part housing 141 and the internal space of the dust collection motor housing 146. The second dust collection flow path 148 may be formed in a direction intersecting the vertical direction. For example, the second dust collection flow path 148 may be formed to be close to the horizontal direction.

In this case, in the present invention, the first dust collection flow path 147 and the second dust collection flow path 148 may be formed at different heights. That is, the first dust collection flow path 147 and the second dust collection flow path 148 may be disposed as layered structures. At least a part of the first dust collection flow path 147 may be disposed above the second dust collection flow path 148.

With this configuration, the plurality of flow paths may be disposed to be close to the horizontal direction, thereby reducing an overall height, and simultaneously, the plurality of flow paths may be stacked, thereby minimizing an overall volume and width of the robot cleaner station 100 in the leftward/rightward direction.

### MOP WASHING PART

Meanwhile, the mop washing part 160 of the robot cleaner station 100 according to the embodiment of the present invention will be described below.

The robot cleaner station 100 according to the embodiment of the present invention may include the mop washing part 160. The mop washing part 160 may supply the washing water to the mop 242 of the robot cleaner 200 coupled to the seating part 120, wash the mop 242, and drain the wastewater produced after the mop 242 is washed.

The mop washing part 160 may include a washing water supply part configured to mix purified water with a liquid containing a detergent and discharge the mixture to an upper side of the washing plate 122. The washing water supply part may include a regulator 161, a mixing chamber 162, the detergent container 163, a branch flow path 164, and washing water nozzles 165.

In this case, the detergent container 163 and the wastewater container 166 may be accommodated in a space formed between the inner wall 124 and the outer wall 111 of the housing. The detergent container 163 may be disposed at a lower side of the space formed between the inner wall 124 and the outer wall 111 of the housing, and the wastewater container 166 may be disposed above the detergent container 163.

The water supply pipe of the kitchen cabinet 2 may be connected to the regulator 161 and adjust a flow rate of the water supplied from the water supply pipe. In addition, a part of the purified water having passed through the regulator 161 may be supplied to the water container 230 of the robot cleaner 200 through the water supply nozzle 123c, and the remaining purified water may be supplied to the mixing chamber 162.

In addition, the liquid containing the detergent stored in the detergent container 163 may be supplied to the mixing chamber 162 by a flow force of a pump. A detailed structure of the detergent container 163 will be described below.

The mixing chamber 162 is provided with a space in which the detergent-containing liquid and the purified water may be introduced and mixed, and the mixing chamber 162 may discharge the washing water in which the detergent and the purified water are mixed. The mixing chamber 162 may be provided with a purified water inlet port 162a, a detergent inlet port 162b, and a branch flow path connection port 162c.

The mixing chamber 162 may be provided in the housing 110 disposed rearward of the seating part 120. In this case, a flow path 161a, into which the purified water introduced from the regulator 161, may be connected to the purified water inlet port 162a. In addition, a flow path 163a, into which the detergent-containing liquid is introduced from the detergent container 163, may be connected to the detergent inlet port 162b. Therefore, the regulator 161 and the pump of the detergent container 163 may operate for a predetermined period of time and allow a preset amount of purified water and detergent to the mixing chamber 162.

Meanwhile, the branch flow path connection port 162c may be connected to the branch flow path 164. The branch flow path 164 may supply the purified water and the detergent-containing washing water to the pair of washing water nozzles 165.

The branch flow path 164 may be provided in a shape having two tube portions diverged from a single tube portion. In this case, an end of one diverged tube portion may be connected to any one of the pair of washing water nozzles 165, and an end of the other diverged tube portion may be connected to the remaining one of the pair of washing water nozzles 165.

The pair of washing water nozzle 165 may be disposed to be spaced apart from each other. In this case, the pair of washing water nozzles 165 may be disposed at positions that are symmetric to each other.

Further, the branch flow path 164 may be connected to the washing water nozzle 165, the washing water may be introduced into the washing water nozzle 165, and the washing water may be discharged to the washing plate 122. The washing water nozzle 165 may discharge the washing water to an upper surface of the washing plate 122 through a washing water discharge port 165a. The washing water discharge port 165a may be opened in a direction in which the washing water discharge port 165a faces an upper surface of the mop 242 seated on the washing plate 122. More specifically, the washing water discharge port 165a formed in the washing water nozzle 165 may discharge the washing water toward a washing protrusion portion 122a of the washing plate 122.

The washing water nozzle 165 may be provided on a nozzle installation wall connected to the coupling wall 123. The washing water nozzle 165 may be positioned at a position higher than an uppermost end of the washing plate 122 so that the washing plate 122 may be detached. Therefore, the washing plate 122 and the washing tub 128 do not collide with the washing water nozzles 165 when the washing plate 122 is detached or the drawer 190 is withdrawn, and a washing water discharge space may be provided between the nozzle installation wall and the washing plate 122.

Further, the washing water nozzles 165 may be disposed at positions spaced apart vertically upward from positions spaced apart from centers of the washing protrusions 122a based on a width direction. Specifically, when the rotation direction of the mop 242 during the process of washing the mop 242 is referred to as one direction, the washing water nozzles 165 may be disposed at positions spaced, in the other direction, apart from the center of the washing protrusion portion 122a based on the width direction. With this configuration, the washing water may flow along the center of the washing protrusion portion 122a based on the width direction.

The detergent container 163 includes a detergent container body 163a, the handle 163b, and detergent container rails 163c.

The detergent container body 163a may provide a space that may store the liquid containing the detergent. For example, the detergent container body 163a may be formed in the shape of a box opened at an upper side thereof.

The handle 163b may be provided forward of the detergent container body 163a. The handle 163b may be configured to be gripped by the user. For example, the handle 163b may be recessed rearward from a front surface of the detergent container body 163a.

With this configuration, when the user grips the handle 163b and pulls the handle 163b forward, the detergent container body 163a may be pulled forward together with the handle 163b and withdrawn. Therefore, according to the present invention, the user may easily pull the detergent container 163 forward, and then the detergent may be supplied.

The detergent container body 163a may be formed with the detergent container rails 163c. The detergent container rail 163c may guide a movement of the detergent container body 163a.

For example, the detergent container rails 163c may be provided in the form of grooves or ribs in the forward/rearward direction on the lateral surfaces of the detergent container body 163a based on the leftward/rightward direction.

With this configuration, in case that the user couples the detergent container 163 to the housing 110, the detergent container 163 may be coupled at an exact position, and the washing water may be prevented from leaking out.

Meanwhile, although not illustrated, rails may be formed on the housing 110 while corresponding to the detergent container rails 163c. The rails may be formed to correspond to shapes and positions of the detergent container rails 163c.

The wastewater container 166 may provide a space that stores the washing water that has been used to wash the mop 242. After the mop 242 is washed, the washing water discharged to the upper surface of the washing plate 122 may flow downward along the inclination of the washing plate 122 and be discharged to the passing holes 122b. The washing water having passed through the passing holes 122b accumulates in the washing tub 128. In addition, the washing water accumulating in the washing tub 128 may be introduced into a wastewater suction flow path 166b through a wastewater inlet port 166a, pass through the wastewater suction flow path 166b, and be introduced into the wastewater container 166. That is, the liquid having passed through the washing plate 122 may flow along the washing tub 128 and be discharged through the wastewater inlet port 166a.

Meanwhile, the wastewater suction flow path 166b is formed in a wastewater suction pipe, the wastewater inlet port 166a is formed at one end of the wastewater suction pipe, and the other end of the wastewater suction pipe communicates with the wastewater container 166. In this case, the wastewater suction pipe may be disposed to pass through a lower side of the mop drying part 170. That is, the wastewater suction flow path 166b may be disposed below the mop drying part 170. In addition, the wastewater suction flow path 166b may be disposed below an outside air supply flow path 171.

The washing water stored in the wastewater container 166 may be discharged to the water drain pipe 25 of the kitchen cabinet 2 through a wastewater discharge flow path 167. One end of the wastewater discharge flow path 167 may be connected to the wastewater container 166, and the other end of the wastewater discharge flow path 167 may be connected to the water drain pipe 25. In this case, the washing water stored in the wastewater container 166 may be allowed to flow through the wastewater discharge flow path 167 by a centrifugal pump (not illustrated) and discharged to the water drain pipe 25.

The wastewater discharge flow path 167 connected to the wastewater container 166 may be connected to the upstream side 25b based on the U-trap 25a of the water drain pipe 25 of the kitchen cabinet 2. This is because an offensive odor or fluid in the water drain pipe 25 may flow reversely to the wastewater discharge flow path 167 in case that the wastewater discharge flow path 167 is connected to the downstream side 25c based on the U-trap 25a of the water drain pipe 25.

In addition, the mop washing part 160 may include a check valve (not illustrated). The check valve may prevent the fluid in the water drain pipe 25 from flowing reversely to the wastewater discharge flow path 167. The check valve may be provided at the other end of the wastewater discharge flow path 167 connected to the water drain pipe 25.

Meanwhile, the mop washing part 160 of the robot cleaner station 100 according to the present invention may further include the steam supply part 169.

The steam supply part 169 may be supplied with purified water through the regulator 161 and heat the water therein. The steam supply part 169 may discharge the heated water to the accommodation space S to sterilize the mop 242. In addition, the steam supply part 169 may generate wet vapor by heating water to a temperature equal to or higher than 100°C. The steam supply part 169 may discharge the generated wet vapor to the accommodation space S to sterilize the mop 242.

The steam supply part 169 may be disposed between the mop drying part 170 and the housing 110 of the dust collection motor. A nozzle of the steam supply part 169 may be disposed on a nozzle coupling wall. A heater coil may be included in the steam supply part 169 and heat water. Therefore, it is possible to stably supply water or humid air maintained at a predetermined temperature.

In addition, the steam supply part 169 may be supplied with water through the water supply pipe, and the water discharged after use may be discharged to the wastewater container 166 through the water drain pipe.

### MOP DRYING PART

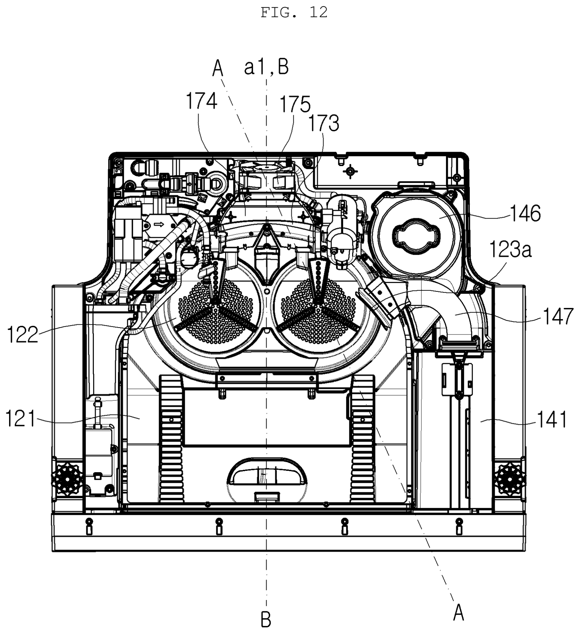

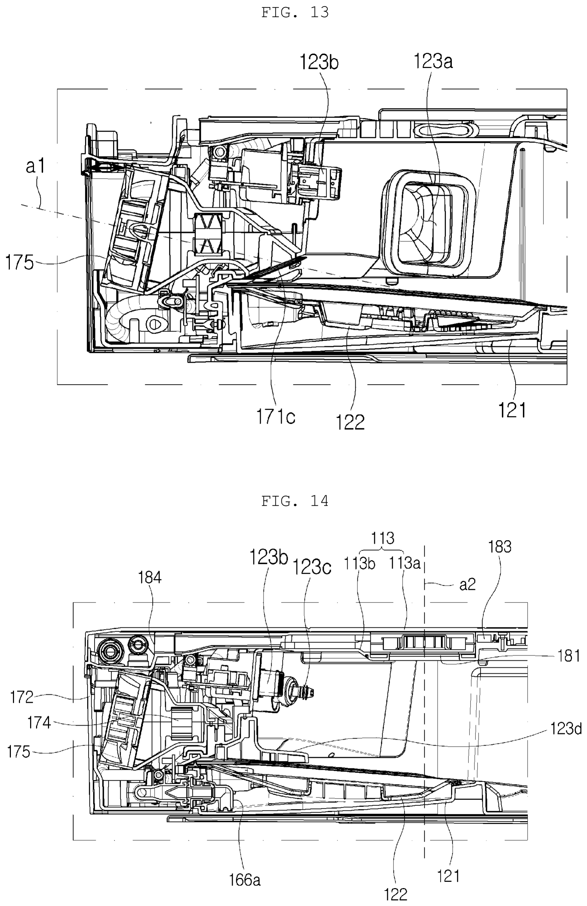

FIG. 12 is a top plan view for explaining the mop drying part of the robot cleaner station according to the embodiment of the present invention. FIG. 13 is a cross-sectional view taken along line A-A in FIG. 12. FIG. 14 is a cross-sectional view taken along line B-B in FIG. 12.

With reference to FIGS. 12 to 14, the robot cleaner station 100 according to the embodiment of the present invention may include the mop drying part 170. In this case, the mop drying part 170 may dry the mop 242 of the robot cleaner 200, which has been washed by the mop washing part 160, or the mop 242 that is wet after a water cleaning process.

The mop drying part 170 may heat air and supply the heated air to the accommodation space S. The mop drying part 170 may include an outside air supply flow path 171, an outside air inlet port 172, the outside air discharge part 173, a heater 174, and a blower fan 175.

The mop drying part 170 is formed with the outside air supply flow path 171. The outside air supply flow path 171 may allow air to flow to the outside air discharge part 173.

The outside air supply flow path 171 may discharge air to the accommodation space. The other side of the outside air supply flow path 171 may communicate with the accommodation space S through the outside air discharge part 173. The outside air supply flow path 171 may discharge air, which is introduced through a gap formed in the front surface, the lower surface, or the rear surface of the housing 110, to the accommodation space S. The blower fan 175 and the heater 174 may be sequentially disposed in the outside air supply flow path 171. A height of the outside air supply flow path 171 may decrease in a direction from the blower fan 175 toward the heater 174.

With this configuration, a flow velocity of air passing through the outside air supply flow path 171 may increase. Therefore, heating efficiency of the heater 174 obtained by the heater 174 may increase.

The outside air inlet port 172 may be formed in the rear surface 111b of the housing 110. The air existing outside the housing 110 may be introduced into the outside air supply flow path 171 through the outside air inlet port 172. In another example, the housing 110 may not include a separate configuration for introducing outside air. That is, air may be introduced through the gap formed in the front surface, the lower surface, or the rear surface of the housing 110. Therefore, the air existing outside the housing 110 may be introduced into the housing 110.

At least a part of the outside air discharge part 173 may be disposed above the washing plate 122. The outside air discharge part 173 may be open in a direction facing the washing plate 122. The outside air discharge part 173 may be provided as a pair of outside air discharge parts 173 opened at lower sides thereof. The pair of outside air discharge parts 173 may be formed to be vertically symmetric with respect to an imaginary axis a1 obtained by extending a rotation axis of a shaft of the blower fan 175.

The outside air discharge part 173 may discharge the air having passed through the outside air supply flow path 171. The outside air discharge part 173 may be disposed forward of the heater 174. The outside air discharge part 173 may discharge the air, which is heated by the heater 174, to the accommodation space S. The air heated by the heater 174 may be divided toward left and right sides and discharged through a pair of outside air discharge ports 173a.

With this configuration, the pair of outside air discharge parts 173 may dry the mops 242 disposed to be vertically symmetric and disposed at the lower side of the robot cleaner 200. In addition, it is possible to prevent a situation in which heat is concentratedly applied to a specific region. Therefore, it is possible to improve efficiency in drying the mop 242.

In particular, the outside air discharge part 173 may have a shape inclined downward toward the front side of the robot cleaner station 100. That is, the outside air discharge part 173 may be provided in a shape inclined downward as the distance from the heater 174 increases. Therefore, an end of the outside air discharge part 173, from which the air is discharged, may be formed to be inclined at a predetermined angle with respect to the ground surface. In this case, the angle may be 90 degrees or less. Therefore, the outside air discharge part 173 may discharge the air in a direction intersecting the direction in which the flow guide surface 122c is formed.

For example, the outside air discharge part 173 may be formed with the outside air outlet port 173a. The outside air discharge port 173a may refer to an open portion of the outside air discharge part 173. The outside air discharge port 173a may discharge air toward the washing plate 122. The outside air discharge part 173 may be open toward the upper surface of the mop 242 in the state in which the mop 242 is seated on the washing plate 122. Therefore, the outside air discharge part 173 may be positioned adjacent to the mop 242 and open downward, such that the air discharged from the outside air discharge part 173 may flow toward the mop 242.

Meanwhile, the outside air discharge port 173a may be penetrated by an imaginary plane C including the axis a1 of the blower fan and an imaginary line connecting left and right points that define a maximum width of the blower fan 175. The blower fan 175 according to the embodiment of the present invention may be disposed in an inclined state. More specifically, a side of the blower fan 175 at which air is discharged may be disposed to be inclined to face the ground surface. In addition, the outside air discharge part 173 may be provided in a shape inclined downward as the distance from the heater 174 increases. Therefore, the plane C including the axis a1 of blower fan may be formed to penetrate the outside air discharge port 173a opened downward.

With this configuration, it is possible to shorten the flow path through which the air discharged from the blower fan 175 reaches the mop 242 through the outside air discharge port 173a. In addition, a velocity of the heated air transmitted to the mop 242 may be increased, thereby improving drying efficiency.

Meanwhile, the outside air discharge part 173 may be provided with a grille configured to guide a discharge direction of air. Therefore, it is possible to prevent the heated air from being concentratedly discharged to a specific position.

The blower fan 175 may be disposed in the outside air supply flow path 171 and blow the air toward the accommodation space S. The blower fan 175 may provide a flow force to the air introduced through the gap in the housing 110.

The blower fan 175 may allow the air to flow to the outside air discharge part 173. When the blower fan 175 operates, the air may be heated by the heater 174 and discharged to the accommodation space S through the outside air discharge part 173. The blower fan 175 may be configured as an axial fan. An air discharge direction of the blower fan 175 may be parallel to the rotation axis of the shaft. An air suction direction of the blower fan 175 may be parallel to the air discharge direction.

With this configuration, the suction direction and the discharge direction of the airflow may be consistent with each other, thereby simplifying the flow path. In addition, it is possible to provide the same flow rate even at a relatively low rotational speed. Therefore, the blower fan 175 having a smaller size may be used in comparison with a case in which the blower fan 175 is configured as a centrifugal fan. Therefore, a space occupied by the blower fan 175 may be minimized, and the number of necessary components may be reduced.

The blower fan 175 may be disposed between the rear surface 111b of the housing 110 and the heater 174. The blower fan 175 may be disposed to have a predetermined gradient with respect to the rear surface 111b. The blower fan 175 may be disposed in the housing 110 so that a side of the blower fan 175 at which air is discharged faces the accommodation space S. The side of the blower fan 175 at which air is discharged may be disposed to be inclined to face the ground surface.

Specifically, the imaginary axis a1 of the blower fan obtained by extending the rotation axis of the shaft of the blower fan 175 may have a predetermined inclination with respect to the ground surface. The axis a1 of the blower fan may have a predetermined inclination with respect to the rear surface 111b. The axis a1 of the blower fan may penetrate the heater 174.

At least a part of the blower fan 175 may be disposed to be spaced apart from the rear surface 111b. The blower fan 175 may be disposed to be inclined at a predetermined angle with respect to the rear surface 111b. Therefore, a distance between an uppermost end of the blower fan 175 and the rear surface 111b may be longer than a distance between a lowermost end of the blower fan 175 and the rear surface 111b.

Meanwhile, when a maximum distance between the blower fan 175 and the rear surface 111b is ensured at a predetermined level or higher, the airflow may be smoothly formed. That is, it is possible to minimize a deterioration in fan motor control (FMC) performance under a maximum output condition of the heater. In particular, in case that a distance between the blower fan 175 and the rear surface 111b is 30 mm, a FMC performance deterioration rate may be maintained at -1.47%.

In case that a maximum distance between the blower fan 175 and the rear surface 111b is set to a distance less than 25 mm, an airflow is not formed smoothly, and the air around the heater 174 is highly likely to stagnate. As a result, a temperature of the heater 174 may increase to 112°C or higher, and a temperature of the side of the blower fan 175 at which air is discharged may also increase to 30°C or higher, which may increase a load on the blower fan 175 and cause a risk of overheating. In addition, a temperature of the outside air discharge port 173a may increase to 73°C or higher, which may increase a likelihood that air is not smoothly discharged.

In case that the maximum distance between the blower fan 175 and the rear surface 111b exceeds 35 mm, a flow of air may be smooth, but the distance from the rear surface 111b is excessively increased, which may cause a deterioration in air suction efficiency of the blower fan 175. Specifically, a flow rate of air is excessively increased by the blower fan 175, which may decrease heat transfer efficiency obtained by the heater 174. In addition, in case that the distance between the blower fan 175 and the rear surface 111b is excessively increased, it is difficult to efficiently utilize the internal space of the housing 110, and a volume occupied by the mop drying part 170 may increase.

Therefore, in the robot cleaner station 100 according to the embodiment of the present invention, the maximum distance between the blower fan 175 and the rear surface 111b may be set to 25 mm or more and 35 mm or less. Therefore, it is possible to ensure airflow stability and maintain a temperature of the heater 174 at an appropriate level. Further, it is possible to optimize the performance of the blower fan 175.

Meanwhile, the blower fan 175 may operate while the steam supply part 169 operates. The blower fan 175 may operate to supply outside air to the accommodation space S while the steam supply part 169 discharges water or wet vapor into the accommodation space S. In case that the blower fan 175 operates to supply outside air, the heater 174 may be maintained in a stopped state. In addition, the operation of the blower fan 175 may be controlled by the controller 300. An air discharge fan 183 may operate based on humidity information measured by a humidity sensor 177.

The heater 174 may be disposed in the outside air supply flow path 171 and heat the air flowing through the outside air supply flow path 171. The heater 174 may heat air. The heater 174 may heat the air discharged through the outside air discharge part 173.

The heater 174 may include a heater housing and a heating element. In this case, the heater housing may be disposed in the outside air supply flow path 171 and have a space in which the heating element may be accommodated. In addition, the heating element may heat air to be introduced into the heater housing. Therefore, the air heated by the heating element may be discharged to the accommodation space S through the outside air discharge part 173 and dry the wet mop 242.

### AIR DISCHARGE PART

The air heated by hot air discharged from the mop drying part 170 may be discharged through the air discharge part 180.

At least a part of the air discharge part 180 may be disposed above the accommodation space S.

The air heated by hot air discharged from the mop drying part 170 may supply heat to the mop 242 of the robot cleaner 200. Therefore, moisture, which is absorbed by the mop 242 and remains in the mop 242, may be vaporized by absorbing heat from the air. The vaporized moisture may flow inside the accommodation space S. Therefore, the air in the accommodation space S may include the vaporized moisture, and a humidity in the accommodation space S may be increased (hereinafter, the air including the vaporized moisture in the accommodation space S may be referred to as "wet vapor").

The air discharge part 180 may discharge the air, which exists in the accommodation space S, to the outside. Specifically, at least a part of the air discharge part 180 may be disposed on the upper cover 113, and the upper cover 113 may cover an upper side of the accommodation space S.

The air heated by hot air discharged from the mop drying part 170 is in a state in which humidity is increased as moisture in the mop 242 is vaporized. Therefore, in case that the robot cleaner station 100 is disposed below the kitchen cabinet 2, wet vapor comes into contact with various types of components, such as the baseboard 26, of the kitchen cabinet 2, which adversely affects the components.

In this case, in the present embodiment, the upper cover 113 covers the upper side of the accommodation space S, and a door 126 covers a front side of the accommodation space S, such that the upper cover 113, together with the door 126, may prevent wet vapor in the accommodation space S from being discharged to the outside, thereby preventing the kitchen cabinet 2 from coming into contact with the wet vapor.

The air discharge part 180 may include an air suction port 181, an air discharge duct 182, and the air discharge fan 183.

The air suction port 181 may communicate with the accommodation space S. The air suction port 181 may be disposed at the upper side of the accommodation space S. The wet vapor in the accommodation space S may be discharged through the air suction port 181. The air in the accommodation space S may be sucked through the air suction port 181.

The air suction port 181 may be disposed to be higher than the robot cleaner 200 from the ground surface in the state in which the robot cleaner 200 is seated on the seating part 120. Therefore, it is possible to improve efficiency in sucking vapor that is raised by convection while the mop is being dried.

For example, the air suction port 181 may be formed in the upper cover 113. In this case, the upper cover 113 may be formed in a shape in which two or more plates are superimposed. Among the two or more plates, the air suction port 181 may be formed in the inner cover 113b, i.e., the lowermost plate, and a flow path, which communicates with the air suction port 181, may be formed between the inner cover 113b and the outer cover 113a, thereby constituting the air discharge duct 182.

In addition, the air suction port 181 may be disposed to be higher than the outside air discharge port 173a based on the ground surface.

In another example, the air suction port 181 may be formed in the air discharge duct 182 provided in the form of a circular or quadrangular pipe, and the air discharge duct 182 may be coupled to the upper cover 113.

With this configuration, when the upper cover 113 is separated, the air discharge duct 182 may be separated together with the upper cover 113. In case that the upper side of the robot cleaner station 100 needs to be opened to perform maintenance or the like, an operator may remove even the air discharge duct 182 by simply lifting the upper cover 113.

The air suction port 181 may be formed in the shape of a hole in the upper cover 113. The air suction port 181 may be provided in the form of a circular hole. An air discharge fan housing may be coupled directly to the corresponding hole portion of the air suction port 181. Therefore, the air introduced through the air suction port 181 may immediately flow to the air discharge fan housing. The air sucked through the air suction port 181 may be introduced into the air discharge fan 183. A distance from the outside air outlet port 171c to the air suction port 181 may be longer than a distance from the outside air outlet port 171c to the mop 242. This is intended to prevent a situation in which the heated air discharged from the outside air outlet port 171c is sucked directly into the air suction port 181 without being sufficiently supplied to the mop 242, which causes waste of energy.

In addition, the air suction port 181 may be disposed to be closer to the door 131 than the outside air outlet port 171c to the door 131. Because the air suction port 181 is disposed at a front upper side of the accommodation space S, a range of a space in which hot air discharged from the outside air discharge part 173 flows is increased, thereby improving efficiency in drying the mop 242. Therefore, the hot air discharged through the outside air discharge part 173 may dry the mop 242 of the robot cleaner 200 while flowing forward, and then the hot air may be discharged to the air suction port 181.

In addition, the air suction port 181 may be disposed above a route along which the robot cleaner 200 moves in the housing 110. Therefore, it is possible to prevent the occurrence of condensation on a wall inside the housing 110.

For example, at least a part of the air suction port 181 may be disposed vertically above a position at which the robot cleaner 200 has a largest width in the leftward/rightward direction in the state in which the robot cleaner 200 is seated on the seating part 120. That is, at least a part of the air suction port 181 may be disposed above a position at which a distance between the robot cleaner 200 and the pair of inner walls 124 is shortest. In this case, at least a part of the air suction port 181 may be disposed forward of the washing plate 122.

Therefore, it is possible to prevent vapor generated during the process of drying the mop 242 from flowing to the front side of the robot cleaner station 100 and to prevent moisture from penetrating into a sensor disposed at the front side of the robot cleaner 200 and causing an erroneous operation.

The air discharge duct 182 may connect the air discharge fan 183 and an air discharge port 184 and communicate with the water drain pipe 25 of the kitchen cabinet 2. The air discharge duct 182 may guide the wet vapor, which is discharged through the air suction port 181, to the water drain pipe 25.

One side of the air discharge duct 182 may be connected to the air suction port 181, and the other side of the air discharge duct 182 may be connected to the air discharge port 184. The air discharge fan 183 may be disposed in the air discharge duct 182. An air discharging flow path may be formed in the air discharge duct 182 and communicate with the air suction port 181.

The air discharge fan 183 may allow the air, which exists in the accommodation space S, to flow to the air suction port 181. A flow of air flowing from the air suction port 181 toward the water drain pipe 25 may be induced. The air discharge fan 183 may generate an airflow in order to suck the wet vapor, which exists in the accommodation space S, into the air suction port 181 and then discharge the wet vapor to the outside through the air discharge duct 182.

The air discharge fan 183 may include an air discharge fan housing, a shaft, a fan motor, and an impeller. A flow path may be formed in the air discharge fan housing and communicate with the air discharge duct 182. When the air discharge fan impeller is rotated by an operation of the air discharge fan motor, the air in the accommodation space S or the housing 110 may be introduced into the air discharge duct 182, pass through an interior of the air discharge fan housing, and then be discharged to the air discharge port 184.

Meanwhile, in the present embodiment, the air discharge fan 183 may be disposed above the route along which the robot cleaner 200 moves in the housing 110. For example, the air discharge fan 183 may be disposed in the upper cover 113. The air discharge fan housing may be disposed between the outer cover 113a and the inner cover 113b included in the upper cover 113.

An imaginary air discharge fan axis a2 obtained by extending the rotation axis of the shaft included in the air discharge fan 183 may be disposed to be perpendicular to the ground surface. That is, an air suction direction of the air discharge fan 183 may be perpendicular to the ground surface.

Meanwhile, in the present embodiment, the air discharge fan 183 may be configured as a centrifugal fan. The air discharge fan 183 may be disposed in the housing 110 so that a side of the air discharge fan 183 at which air is sucked may be disposed to face the accommodation space S. An air discharge direction of the air discharge fan 183 may be perpendicular to the rotation axis of the shaft. The air suction direction of the air discharge fan 183 may be perpendicular to the air discharge direction. When the air discharge fan 183 operates, the air in the accommodation space S may be introduced into the air suction port 181. The air introduced into the air suction port 181 may be discharged to the water drain pipe 25.

In this case, the air discharge port 184 may be formed in a shape opened toward a rear side of the housing 110. Condensate water, which is generated by the wet vapor discharged through the air discharge port 184, may be collected along an inclined surface formed on a fitting coupling portion 114 of the housing 110. The air discharged from the air discharge port 184 may be diffused into the outside air.

Meanwhile, the air discharge fan 183 may operate while the steam supply part 169 operates. The air discharge fan 183 may operate to adjust humidity in the robot cleaner station 100 while the steam supply part 169 discharges water or wet vapor into the accommodation space S. The operation of the air discharge fan 183 may be controlled by the controller 300. An air discharge fan 183 may operate based on humidity information measured by a humidity sensor 177.

### DRAWER

In case that the charging stand for the robot cleaner is disposed below the kitchen cabinet, the exposure to the outside is minimized, which may provide an interior effect. However, there is a limitation in that it may be difficult for the user to extract and repair the robot cleaner and the charging stand in case that the robot cleaner is broken down in the state in which the robot cleaner is placed below the kitchen cabinet or in case that the charging stand for the robot cleaner is broken down. In order to cope with the above-mentioned limitation, in the present invention, the robot cleaner station 100 may be further equipped with the drawer 190.

The robot cleaner station 100 according to the embodiment of the present invention may further include the drawer 190 configured to be withdrawn from the housing 110.

The drawer 190 may move relative to the housing 110. For example, the housing 110 may be fixedly coupled to the kitchen cabinet 2, and the drawer 190 may be withdrawn forward from the housing 110.

In this case, the drawer 190 may be withdrawn in the state in which the seating part 120 is provided in the drawer 190. With this configuration, when the drawer 190 is withdrawn, the seating part 120 and/or the robot cleaner 200 may be withdrawn from the kitchen cabinet 2 to the outside.

In this case, the upper cover 113 may be exposed to the outside when the drawer 190 is withdrawn from the housing 110 in the state in which the door 131 closes the inlet/outlet 127. In this case, when the upper cover 113 is disassembled, the robot cleaner 200 may be exposed to the outside.

Therefore, according to the present embodiment, in case that the robot cleaner station 100 is required to be subjected to maintenance such as repair or cleaning, the user may easily withdraw the seating part 120 and/or the robot cleaner 200 by means of the drawer 190 and expose the constituent elements in the robot cleaner station 100 or the robot cleaner 200.

Meanwhile, the drawer 190 according to the embodiment of the present invention may be withdrawn in the state in which the dust collection part 140 is provided in the drawer 190. In this case, a withdrawal direction of the drawer 190 may be parallel to a withdrawal direction of the dust bag drawer 144.

In addition, the drawer 190 according to the embodiment of the present invention may be withdrawn together with the mop washing part 160. Specifically, the drawer 190 may be withdrawn together with the detergent container 163. In this case, a withdrawal direction of the drawer 190 may be parallel to a withdrawal direction of the detergent container 163.

With this configuration, in the robot cleaner station 100 according to the embodiment of the present invention, the withdrawal directions of all the drawer 190, the dust bag drawer 144, and the detergent container 163 may be parallel to one another.

Therefore, the user may easily recognize the withdrawal directions of the constituent elements of the robot cleaner station 100 of the present invention and easily withdraw, repair, and maintain the constituent elements of the robot cleaner station 100.

The drawer 190 includes drawer sidewalls 191, fitting parts 192, and drawer rails 193.

The drawer sidewalls 191 are configured to be movable relative to the outer wall surfaces of the housing 110. For example, the pair of drawer sidewalls 191 may be disposed to face the pair of outer walls of the housing 110.

In this case, the pair of drawer sidewalls 191 may be disposed to be closer to the inside of the robot cleaner station 100 than the pair of outer walls of the housing 110. That is, the pair of drawer sidewalls 191 may be disposed to be closer to the seating part 120 than the pair of outer walls of the housing 110.

Meanwhile, the dust collection part 140 and/or the mop washing part 160 may be disposed between the drawer sidewall 191 and the seating part 120.

With this configuration, the dust collection part 140 and the mop washing part 160 may be disposed by utilizing a minimum horizontal space.

The drawer rail 193 may be disposed on the drawer sidewall 191 and guide a movement of the drawer sidewall 191. The drawer rail 193 may be fixedly coupled to or integrated with the drawer sidewall 191. The drawer rail 193 may be coupled to the rail installed on the outer wall 111 of the housing 110 and guide a movement route of the drawer sidewall 191. Meanwhile, the present invention discloses that the rails are provided on the drawer 190 and the housing 110. However, the present invention is not necessarily limited to the rail shape. A roller, a guide groove, a guide rib, or the like, which may be substituted for the rail, may be included.

### CONTROL CONFIGURATION

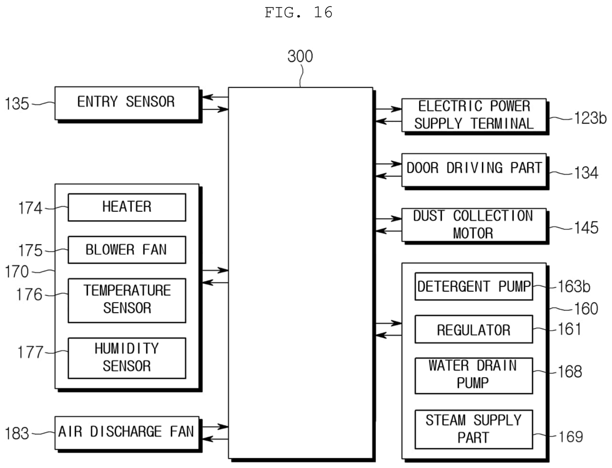

FIG. 16 is a block diagram for explaining a control configuration of the cleaner station according to the embodiment of the present invention.

The control configuration of the robot cleaner station 100 of the present invention will be described below with reference to FIG. 16.

The cleaner station 100 according to the embodiment of the present invention further include a controller 300 configured to control the seating part 120, the dust collection motor 145, the mop washing part 160, and the mop drying part 170.

The controller 300 may include a printed circuit board and elements mounted on the printed circuit board.

The controller 300 may receive a signal from the entry sensor 135 and control the door driving part 134.

The controller 300 may detect the approach of the robot cleaner 200 and control the door driving part 134 to rotate the door 131. Specifically, the controller 300 may detect, by means of the entry sensor 135, whether the robot cleaner 200 enters. Specifically, the controller 300 may open the inlet/outlet 127 by rotating the door 131 when a distance between the robot cleaner 200 and the door 131 is shorter than a preset distance. In addition, the controller 300 may close the inlet/outlet 127 by rotating the door 131 when the robot cleaner 200 is coupled to the seating part 120.

When electric power is supplied to the battery of the robot cleaner 200 from the electric power supply terminal 123b, the controller 300 may determine that the robot cleaner 200 is coupled to the seating part 120.

The controller 300 may open the dust bin door 222 of the robot cleaner 200. The controller 300 may operate the dust collection motor 145 to suck dust from the inside of the dust bin 220 of the robot cleaner 200.

Meanwhile, the robot cleaner station 100 according to the embodiment of the present invention may include a memory (not illustrated). The memory may include various data for operating or driving the robot cleaner station 100.

Meanwhile, the robot cleaner station 100 according to the embodiment of the present invention may include a communication part (not illustrated). The communication part may support wireless communication with other devices, such as the robot cleaner 200 or a terminal (not illustrated), existing outside the robot cleaner station 100. As a wireless communication module for wireless communication support, a near-field communication module or a far-field communication module may be provided.

For example, a short-range communication may be Bluetooth communication, near field communication (NFC) communication, or the like.

For example, the far-field communication may be wireless LAN (WLAN), digital living network alliance (DLNA), wireless broadband (Wibro), world interoperability for microwave access (Wimax), global system for mobile communication (GSM), code division multi-access (CDMA), code division multi-access 2000 (CDMA 2000), enhanced voice-data optimized or enhanced voice-data only (EV-DO), wideband CDMA (WCDMA), high-speed downlink packet access (HSDPA), high-speed uplink packet access (HSUPA), IEEE 802.16, long term evolution (LTE), long term evolution-advanced (LTEA), wireless mobile broadband service (WMBS), Bluetooth low energy (BLE), Zigbee, radio frequency (RF), long range LoRa, and the like.

The controller 300 may control the mop washing part 160.

Specifically, the controller 300 may control a detergent pump 163b. The controller 300 may operate the detergent pump 163b to discharge the detergent, which is stored in the detergent container 163, to the mop 242.

In addition, the controller 300 may control the regulator 161. The controller 300 may operate the regulator 161 to adjust the amount of purified water to be discharged to the mop 242.

In addition, the controller 300 may control a water drain pump 168. The controller 300 may operate the water drain pump 168 to drain wastewater produced after the mop 242 is washed.

The controller 300 may control the mop drying part 170.

Specifically, the controller 300 may control the heater 174. The controller 300 may operate the heater 174 to heat air to be discharged to the mop 242.

In addition, the controller 300 may control the blower fan 175. The controller 300 may operate the blower fan 175 to discharge air to the mop 242.

The controller 300 may control the air discharge part 173.

In addition, the controller 300 may control the air discharge fan 183. The controller 300 may operate the air discharge fan 183 to discharge air to the outside after the mop 242 is dried.

In addition, the controller 300 may receive a signal from a temperature sensor 176. The controller 300 may measure a temperature of air in the housing 110 based on temperature information received from the temperature sensor 176. Further, the controller 300 may sterilize bacteria present in the mop 242 by controlling the operation of the heater 174 based on temperature information received from the temperature sensor 176.

The controller 300 may move the rotary plate 241 to the lower side of the robot cleaner 200. The controller 300 may rotate the rotary plate 241.

The controller 300 may control the steam supply part 169.

The controller 300 may supply water into the steam supply part 169 and then heat the water to a temperature of 55°C or more and less than 70°C. Alternatively, the controller 300 may heat water inside the steam supply part 169 to a temperature of 100°C or more. In addition, the controller 300 may discharge water or wet vapor from the steam supply part 169 into the accommodation space S.

The controller 300 may receive information about internal humidity of the robot cleaner station 100 from the humidity sensor 177. Further, the controller 300 may adjust the internal humidity by controlling the air discharge fan 183, the blower fan 175, or the door driving part 134 based on the humidity information received from the humidity sensor 177.

### CONTROL METHOD

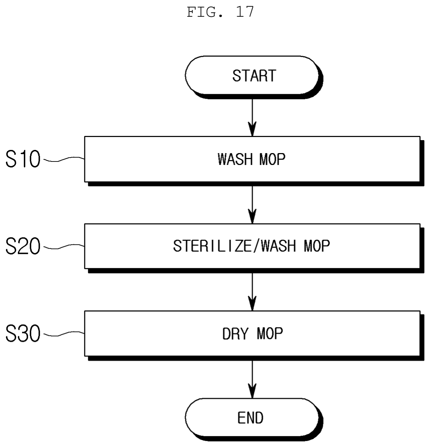

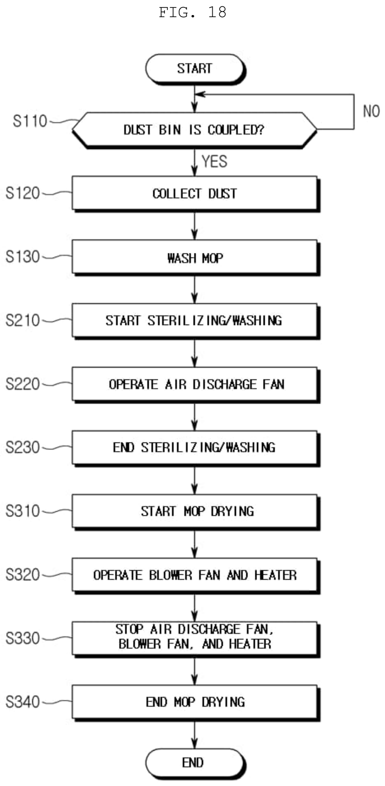

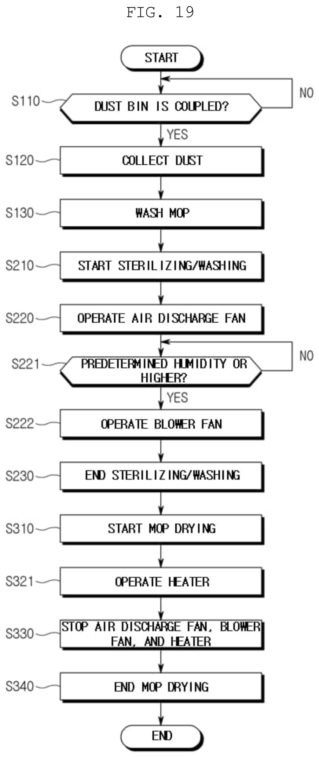

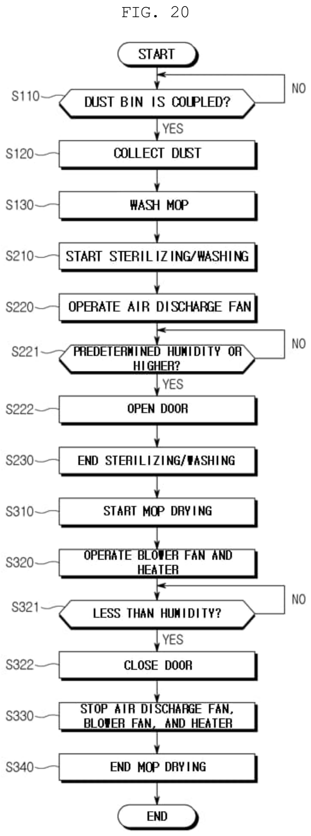

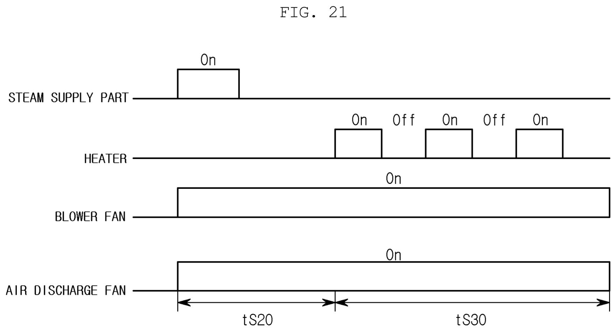

FIGS. 17 to 19 are flowcharts for explaining a method of controlling the robot cleaner station according to the embodiment of the present invention. FIG. 20 is a flowchart for explaining a method of controlling the robot cleaner station according to another embodiment of the present invention. FIG. 21 is a view for explaining operations of the steam supply part, the heater, the blower fan, and the air discharge fan over time in the method of controlling the robot cleaner station according to the embodiment of the present invention.

The method of controlling the robot cleaner station 100 of the present invention will be described below with reference to FIGS. 17 to 21.

First, the method of controlling the robot cleaner station of the present invention may include a dust collection step S120.

Specifically, in the dust collection step S120, an operation of emptying the dust bin 220 of the robot cleaner 200 may be performed in case that it is identified that the robot cleaner 200 is coupled to the robot cleaner station 100.

The controller 300 may receive a signal generated from a coupling sensor 125 and identify whether the robot cleaner 200 is seated on the seating part 120.

Meanwhile, the controller 300 may identify whether the robot cleaner 200 is coupled at an exact position by determining whether a charging part supplies power to the battery of the robot cleaner 200.

Therefore, the controller 300 may receive a signal, which indicates that the robot cleaner 200 is coupled, from the coupling sensor 125 and identify whether the charging part supplies power to the battery, thereby identifying whether the robot cleaner 200 is seated on the seating part 120 of the cleaner station 100.

In the dust collection step S120, the dust bin door 222 of the robot cleaner 200 may be opened when the robot cleaner 200 is seated in the robot cleaner station 100. When the dust bin door 222 is opened, the dust collection motor 145 may operate to collect dust from the interior of the dust bin 220.

Specifically, when the controller 300 receives a signal, which indicates that the dust bin door 222 is opened, from a first cover opening detecting part 155fa, the controller 300 may operate the dust collection motor 145.

In the dust collection step S120, the dust inside the dust bin 220 may pass through the dust bin, the hole 123a, and the dust collection flow paths 147 and 148 and collected in the dust collection part 140. Therefore, the user may remove the dust inside the dust bin 220 without a separate manipulation, and as a result, it is possible to provide convenience for the user.

The method of controlling the robot cleaner station of the present invention may include a mop washing step S10, a sterilizing/washing step S20, and a mop drying step S30.

In the mop washing step S10, the washing water may be supplied to the mop 242 of the robot cleaner 200 to wash the mop 242.

Specifically, the controller 300 may start the mop washing step S10 when a signal, which indicates that the dust collection step S120 ends, is generated. The controller 300 may operate the washing water supply part to supply the washing water.

The washing water supply part may be configured to spray the washing water directly onto the mop 242 or onto the washing plate 122 to deliver the washing water when the mop 242 comes into contact with the washing plate 122.

In addition, when the controller 300 recognizes that the mop washing step S10 is initiated, the controller 300 may move the rotary plate 241 of the robot cleaner 200 downward. The rotary plate 241 may be configured to be movable upward or downward. When the rotary plate 241 moves downward, at least a part of the mop 242 attached to the lower side of the rotary plate 241 may be disposed to be in contact with the washing plate 122.

Thereafter, the controller 300 may rotate the rotary plate 241. In this case, the mop 242 may be washed by friction while rotating in a state in which the mop 242 is in contact with the washing plate 122.

The sterilizing/washing step S20 may discharge heated water or wet vapor to the mop 242 to sterilize the mop 242.

The sterilizing/washing step S20 may include a sterilizing/washing starting step S210, an air discharge fan operating step S220, and a sterilizing/washing ending step S230.

In the sterilizing/washing starting step S210, the sterilizing/washing step S20 may be performed when the mop washing step S10 ends. Specifically, when the controller 300 receives a signal that indicates that the mop washing step S10 ends, the controller 300 may perform the sterilizing/washing step S20.

The controller 300 may supply water into the steam supply part 169 and then heat the water to a temperature of 55°C or more and less than 70°C. When the water is heated within a preset temperature range, the controller 300 may discharge the heated water to the mop 242.

Alternatively, the controller 300 may operate the steam supply part 169 to heat water inside the steam supply part 169 to a high temperature of 100°C or more. When vapor is generated by heating water, the controller 300 may discharge the generated vapor toward the mop 242.

Meanwhile, in some instances, the controller 300 may operate to discharge the heated water for a predetermined period of time first and then additionally discharge the vapor. For example, water heated to a temperature of 55°C or higher and less than 70°C is first sprayed onto the mop 242 to soften contaminants, and steam at 100°C or higher is then continuously discharged, such that the remaining contaminants may be removed and a sterilization effect may be enhanced.

Such a method may be automatically set by the controller 300 depending on a contaminated state of the mop 242 or a cleaning mode and also be directly selected by the user as necessary.

Accordingly, contaminants attached to the mop 242 may be effectively removed, and bacteria and harmful microorganisms may be reduced through a sterilization action by high-temperature steam, thereby enabling hygienic cleaning.

In the air discharge fan operating step S220, the controller 300 may operate the air discharge fan 183. Specifically, the controller 300 may discharge inside air by operating the air discharge fan 183 while the sterilizing/washing step S20 is performed.

For example, when the controller 300 receives a signal that indicates that the mop washing step S10 ends, the controller 300 may operate the air discharge fan 183. Therefore, the air discharge fan 183 may operate while the sterilizing/washing step S20 is performed.

In another example, the controller 300 may identify information about internal humidity of the robot cleaner station 100 from the humidity sensor 177 while the sterilizing/washing step S20 is performed. In this case, when the internal humidity is measured to be 70% or more, the controller 300 may operate the air discharge fan 183 to discharge inside air.

In contrast, when the internal humidity is less than a predetermined value, the sterilizing/washing step S20 may be continuously performed without operating the air discharge fan 183.

Meanwhile, the sterilizing/washing step S20 of the control method according to the embodiment of the present invention may further include a humidity identifying step S221 and a blower fan operating step S222.

In the humidity identifying step S221, the controller 300 may identify the internal humidity by receiving a signal from the humidity sensor 177. Specifically, when the controller 300 receives a signal, which indicates that the internal humidity of the robot cleaner station 100 is a predetermined value or more, from the humidity sensor 177, the controller 300 may determine that outside air is required to be introduced.

For example, when the internal humidity is measured to be 70% or more, a state with high humidity may be determined, and the blower fan operating step S222, which is the next step, may be performed. In contrast, in case that the internal humidity is less than a preset reference value, the process may not proceed to the blower fan operating step S222. In this case, when a preset sterilizing/washing time tS20 has elapsed, the process may proceed to the sterilizing/washing ending step S230.

In the blower fan operating step S222, the controller 300 may operate the blower fan 175 depending on a result of the humidity identifying step S221. Specifically, in case that the controller 300 receives a signal that indicates that the internal humidity is 70% or more, the controller 300 may operate the blower fan 175 to introduce outside air into the robot cleaner station 100. Therefore, the circulation of inside air may be promoted, and moisture may be removed.

The air discharge fan 183 and the blower fan 175 may operate simultaneously or individually depending on an internal humidity state, such that moisture inside the robot cleaner station 100 may be removed, and growth of mold and bacteria may be suppressed, thereby maintaining a hygienic state.

Meanwhile, according to another embodiment of the present invention, a door opening step S222' may be performed after the humidity identifying step S221.

In the humidity identifying step S221, the controller 300 may identify a humidity state by receiving internal humidity information from the humidity sensor 177. Specifically, when the controller 300 receives a signal, which indicates that the internal humidity is a predetermined value or more, from the humidity sensor 177, the controller 300 may determine that inside air is required to be introduced, and the controller 300 may perform the door opening step S222' that is the next step.

In the door opening step S222', the controller 300 may open the door 131 depending on a result of the humidity identifying step S221. For example, when the controller 300 receives a signal, which indicates that the internal humidity is 70% or more, from the humidity sensor 177, the controller 300 may open the door 131 by operating the door driving part 134. When the door 131 is opened, outside air may be introduced into the robot cleaner station 100. In contrast, in case that the internal humidity is less than a preset reference value, the process may not proceed to the door opening step S222'. In this case, when the preset sterilizing/washing time tS20 has elapsed, the process may proceed to the sterilizing/washing ending step S230.

Meanwhile, in order to introduce outside air, the process of opening the door 131 may be performed together with the operation of the blower fan 175.

In the sterilizing/washing ending step S230, when the preset sterilizing/washing time tS20 has elapsed, the controller 300 may determine that the sterilizing/washing step S20 ends. The sterilizing/washing time tS20 may be determined in accordance with a time preset by the user. Alternatively, an appropriate time may be inputted in advance to the controller 300. In this case, the controller 300 may stop the steam supply part 169 and end the sterilizing/washing step S20 (S230).

In the mop drying step S30, the blower fan 175, which is configured to discharge air toward the mop 242 of the robot cleaner 200, may operate to dry the mop.

The mop drying step S30 may include a mop drying starting step S310, a blower fan/heater operating step S320, an air discharge fan/blower fan/heater stopping step S330, and a mop drying ending step S340.

In the mop drying starting step S310, when the controller 300 receives a signal that indicates that the sterilizing/washing step S20 ends, the controller 300 may start the mop drying step. Thereafter, the process may proceed to the next step, and the blower fan/heater operating step S320 may be performed.

In the blower fan/heater operating step S320, the controller 300 may operate the blower fan 175. Meanwhile, in case that the blower fan 175 already operates in the sterilizing/washing step S20 in accordance with the humidity condition, the controller 300 may maintain the operation of the blower fan 175.

In addition, the controller 300 may operate the heater 174. Therefore, the air heated by the heater 174 may be discharged to the mop 242 through the blower fan 175.

Meanwhile, the controller 300 may operate the air discharge fan 183. In case that the air discharge fan 183 already operates, the controller 300 may maintain the operation of the air discharge fan 183.

The mop drying step S30 of the method of controlling the robot cleaner station according to another embodiment of the present invention may further include a humidity identifying step S321 and a door closing step S322.

In the humidity identifying step S321, the controller 300 may identify humidity by receiving internal humidity information from the humidity sensor 177. Specifically, when the controller 300 receives a signal that indicates that the internal humidity is less than a predetermined value, the controller 300 may determine that outside air is not required to be introduced any further, and the controller 300 may perform the door closing step S322 that is the next step. For example, in case that the internal humidity is measured to be less than 35%, the controller 300 may perform the door closing step S322.

In the door closing step S322, the controller 300 may close the door 131 depending on a result of the humidity identifying step S321. Specifically, when the controller 300 may receive a signal, which indicates that the internal humidity is less than 35%, from the humidity sensor 177, the controller 300 may close the door 131 by operating the door driving part 134. Therefore, in case that the internal humidity is maintained at a predetermined level or lower, unnecessary introduction of outside air may be blocked, thereby stably maintaining an internal environment.

In contrast, in case that the internal humidity is a preset reference value or more, the process does not proceed to the door closing step S322. In this case, when a preset mop drying time tS30 has elapsed, the process may proceed to the air discharge fan/blower fan/heater stopping step S330 that is the next step.

In the air discharge fan/blower fan/heater stopping step S330, when the controller 300 determines that the predetermined drying time tS30 has elapsed, the controller 300 may stop the operations of the constituent elements used for the mop drying process. Specifically, the controller 300 may stop the heater 174. The controller 300 may stop the air discharge fan 183.

The controller 300 may stop the blower fan 175. Meanwhile, in a state in which the blower fan 175 is already stopped in the sterilizing/washing step S20 in accordance with the humidity condition, the controller 300 may maintain the stopped state of the blower fan 175.

In the method of controlling the robot cleaner station according to another embodiment of the present invention, the controller 300 may close the door 131 when the controller 300 determines that the mop drying time tS30 has elapsed. In this case, in a state in which the door 131 is already closed, the controller 300 may maintain the corresponding closed state.

In the mop drying ending step S340, the controller 300 may end the mop drying step S30 in case that a predetermined time has elapsed. The mop drying time tS30 may be determined depending on a value preset by the user, or a predetermined time on the system may be set as a value. The mop drying step S30 may end when the preset time has elapsed.

Alternatively, the controller 300 may measure humidity of the mop, and the controller 300 may end the mop drying step S30 in case that the humidity of the mop decreases to a predetermined reference value or less.

An operational sequence of the steam supply part 169, the heater 174, the blower fan 175, and the air discharge fan 183 according to the method of controlling the robot cleaner station according to the embodiment of the present invention will be described below with reference to FIG. 21.

In the sterilizing/washing step S20, the controller 300 may heat water by operating the steam supply part 169 and then discharge the heated water or vapor to the mop 242. In this case, the controller 300 may maintain the operation of the steam supply part 169 for a predetermined period of time, and then the controller 300 may stop the operation of the steam supply part 169 when a preset temperature or time condition is satisfied. A period of time for which the steam supply part 169 operates may be shorter than the sterilizing/washing time tS20.

When the sterilizing/washing step S20 is started, the air discharge fan 183 may operate to discharge wet vapor. When the sterilizing/washing step S20 is started, the blower fan 175 may operate to introduce outside air. The controller 300 may maintain the operation of the air discharge fan 183 or the blower fan 175 for the sterilizing/washing time tS20.

In contrast, although not illustrated, the operations of the air discharge fan 183 and the blower fan 175 may be adjusted in accordance with the humidity condition. For example, the controller 300 may receive internal humidity information from the humidity sensor 177 while the sterilizing/washing step S20 is performed, and the controller 300 may determine whether to operate the air discharge fan 183 and the blower fan 175 based on the internal humidity information. If the internal humidity increases to a predetermined value or more, the controller 300 may operate the air discharge fan 183 and the blower fan 175 to discharge moisture to the outside and circulate inside air. On the contrary, when the internal humidity decreases to less than a preset reference value, the operation of the air discharge fan 183 or the blower fan 175 may be stopped, or only the blower fan 175 may operate to adjust the introduction of outside air.

In the mop drying step S30, the controller 300 may generate heated air by operating the heater 174. In this case, the generated heated air may be discharged toward the mop 242 by the blower fan 175. During this process, the air discharge fan 183 may serve to adjust the internal humidity by discharging inside air to the outside. The controller 300 may maintain the operation of the heater 174, the air discharge fan 183, or the blower fan 175 for the mop drying time tS30.

In another example, the heater 174 may be controlled to be repeatedly turned on and off at a predetermined interval in the state of continuously operating for the mop drying time tS30. The controller 300 may stop the heater 174 when a predetermined time has elapsed after the mop drying starting step, and thereafter the controller 300 may operate the heater 174 again when a predetermined time has elapsed. In this manner, the controller 300 may repeatedly turn on and off the heater two to three times.

This method may efficiently maintain the drying effect while preventing excessive heat generation.

While the present invention has been described with reference to the specific embodiments, the specific embodiments are only for specifically explaining the present invention, and the present invention is not limited to the specific embodiments. It is apparent that the present invention may be modified or altered by those skilled in the art without departing from the technical spirit of the present invention.

All the simple modifications or alterations to the present invention fall within the scope of the present invention, and the specific protection scope of the present invention will be defined by the appended claims.

### DESCRIPTION OF REFERENCE NUMERALS

1: Robot cleaner station
2: Kitchen cabinet
100: Robot cleaner station
110: Housing
120: Seating part
122: Washing plate
128: Washing tub
130: Door part
131: Door
140: Dust collection part
141: Dust collection part housing
144: Dust bag drawer
145: Dust collection motor
160: Mop washing part
169: Steam supply part
170: Mop drying part
171: Outside air supply flow path
172: Outside air inlet port
173: Outside air discharge part
174: Heater
175: Blower fan
180: Air discharge part
181: Air suction port
182: Air discharge duct
183: Air discharge fan
184: Air discharge port
190: Drawer
200: Robot cleaner
300: Controller

## Claims

1. A robot cleaner station (100) comprising:
a housing (110) comprising an upper cover (113);
a base (121) accommodated in the housing (110) and disposed below a robot cleaner (200);
an accommodation space (S) formed between the base (121) and the upper cover (113) and configured to accommodate the robot cleaner (200);
a mop washing part (160) configured to wash a mop (242) of the robot cleaner (200); and
a mop drying part (170) configured to dry the mop (242) by discharging air to the mop (242),
**characterized in that** the mop washing part (160) comprises a steam supply part (169) configured to heat water and supply hot water or wet vapor to the accommodation space (S),
wherein the mop drying part (170) comprises a blower fan (175) configured to generate an airflow and discharge the air into the accommodation space (S), and
wherein the blower fan (175) is configured to operate while the steam supply part (169) operates.

2. The robot cleaner station (100) of claim 1, comprising:
an air discharge part (180) configured to discharge air from the accommodation space (S),
wherein the air discharge part (180) comprises:
an air suction port (181) configured to communicate with the accommodation space (S) and suck air; and
an air discharge fan (183) configured to provide a flow force to the air sucked through the air suction port (181), and
wherein the air discharge fan (183) is configured to operate while the steam supply part (169) operates.

3. The robot cleaner station (100) of claim 2,
wherein the blower fan (175) is disposed in the housing (110) so that a side of the blower fan (175) at which air is discharged is directed toward the accommodation space (S), and
wherein a side of the air discharge fan (183) at which air is sucked is disposed to be directed toward the accommodation space (S).

4. The robot cleaner station (100) of any one of claims 1 to 3,
wherein the housing (110) comprises a door (131) configured to open or close an inlet/outlet (127) through which the robot cleaner (200) enters or exits, and
wherein the door (131) is configured to be opened while the steam supply part (169) operates.

5. The robot cleaner station (100) of any one of claims 2 to 4, further comprising:
a humidity sensor configured to measure humidity in the housing (110),
wherein the air discharge fan (183) operates when the humidity measured by the humidity sensor is a predetermined reference value or more.
and/or
wherein the blower fan (175) operates when the humidity measured by the humidity sensor is a predetermined reference value or more.

6. The robot cleaner station (100) of claim 4, further comprising:
a humidity sensor configured to measure humidity in the housing (110),
wherein the door (131) is opened when the humidity measured by the humidity sensor is a predetermined reference value or more.

7. A method of controlling a robot cleaner station (100), the method comprising:
a mop washing step of washing a mop (242) of a robot cleaner (200) by supplying washing water to the mop (242) in a state in which the robot cleaner (200) is coupled to the robot cleaner station (100);
a sterilizing/washing step of sterilizing the mop (242) by discharging heated water or wet vapor to the mop (242); and
a mop drying step of drying the mop (242) by operating a blower fan (175) configured to discharge air toward the mop (242),
wherein the blower fan (175) operates in the sterilizing/washing step.

8. The method of claim 7, wherein an air discharge fan (183) configured to discharge air from an interior of the robot cleaner station (100) operates in the sterilizing/washing step.

9. The method of claim 7 or 8, wherein air is introduced when humidity in the robot cleaner station (100) is equal to or greater than a predetermined reference value.

10. The method of claim 7, 8, or 9, wherein a door (131) of the robot cleaner station (100) is opened when humidity in the robot cleaner station (100) is equal to or greater than a predetermined reference value.

11. The method of claim 7, 8, 9, or 10, wherein the blower fan (175) operates when humidity in the robot cleaner station (100) is equal to or greater than a predetermined reference value.

12. The method of any one of claims 9 to 11, wherein the introduction of the air is stopped when the humidity in the robot cleaner station (100) is less than the predetermined reference value.

13. The method of any one of claims 10 to 12, wherein the door (131) is closed when the humidity in the robot cleaner station (100) is less than the predetermined reference value.

14. The method of any one of claims 11 to 13, wherein the operation of the blower fan (175) is stopped when the mop drying step ends.

15. The method of claim 8, wherein the operation of the air discharge fan (183) is stopped when the mop drying step ends.
